(19) Europäisches Patentamt
European Patent Office
Office européen des brevets

(11) Publication number: 0 213 484
B1

(12) **EUROPEAN PATENT SPECIFICATION**

(45) Date of publication of patent specification: 30.01.91

(51) Int. Cl.$^5$: C 07 D 405/06, C 08 K 5/34

(21) Application number: 86111175.5

(22) Date of filing: 12.08.86

(54) **Alpha-Hydroxy-beta-2,2,6,6-tetramethyl piperidyl ethylene oxiranes as light and heat stabilizers for polyvinyl chloride resins.**

(30) Priority: 13.08.85 JP 178322/85

(43) Date of publication of application: 11.03.87 Bulletin 87/11

(45) Publication of the grant of the patent: 30.01.91 Bulletin 91/05

(84) Designated Contracting States: BE CH DE FR GB LI NL

(56) References cited:
EP-A-0 097 616
FR-A-2 331 556
US-A-3 219 612
US-A-4 460 725

(73) Proprietor: ADEKA ARGUS CHEMICAL CO., Ltd.
5-2-13 Shirahata, Urawa City
Saitama Prefecture (JP)

(72) Inventor: Kitsukawa, Kazumi
3-12-20-306 Hikonari
Misato City Saitama (JP)
Inventor: Suzuki, Shigeru
1-139 Miyauchi
Kitamoto City Saitama (JP)
Inventor: Nakazawa, Kenji
Miyabi-Corpo 305 5-15-9 Uchiya
Urawa Saitama (JP)
Inventor: Ishizuka, Hidehiro
Corpo Machida 202 4-18-5 Tsuji
Urawa Saitama (JP)

(74) Representative: Weinhold, Peter, Dr. et al
Patentanwälte Dipl.-Ing. G. Dannenberg Dr. P. Weinhold Dr. D. Gudel Dipl.-Ing. S. Schubert Dr. P. Barz Siegfriedstrasse 8
D-8000 München 40 (DE)

**The file contains technical information submitted after the application was filed and not included in this specification**

EP 0 213 484 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Polyvinyl chloride resins have a relatively low resistance to deterioration when exposed to elevated temperatures and to ultraviolet light, and various types of compounds have been combined therewith to improve their resistance to such deterioration. Metal compounds, such as lead carboxylates and other polyvalent metal salts of carboxylic acids, as well as organotin compounds, have been widely used, alone and in combination with other compounds such as epoxy compounds, aminocrotonates, diphenyl urea, alpha-phenyl indole, orthoesters, and dihydropyridines. The epoxy compounds are known not only as stabilizers but also as plasticizers. Epoxides such as Bisphenol A, diglycidylether, epoxidized unsaturated fatty acid esters, trioglycidylisocyanurate and epoxidized polybutadiene have been used.

The 2,2,6,6-tetramethylpiperidine compounds have also been combined with polyvinyl chloride resins to improve their resistance to ultraviolet light, and epoxy compounds have been used with 2,2,6,6-tetramethyl piperidyl compounds for this purpose. However, some 2,2,6,6-tetramethyl piperidyl compounds impart a color to the polyvinyl chloride resin when heated.

Soma, Moromura, Yoshioka and Kurumada U.S. patent No. 4,371,644, patented February 1, 1983, provide piperidine derivatives having the general formula:

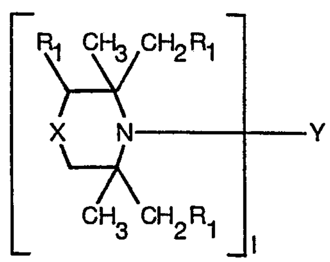

in which:

$R_1$ represents a hydrogen atom or a methyl group;

X represents one of the groups of formulae

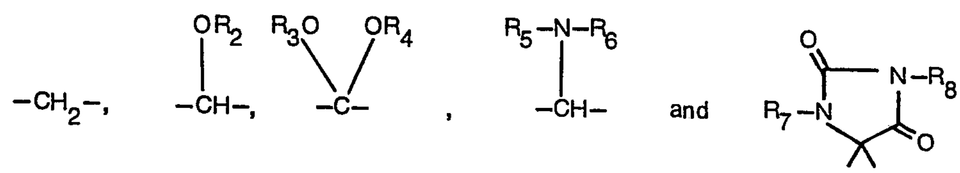

and

Y represents one of the groups of formula $—CH_2CH(OZ)CH_2—[OCH_2CH(OZ)CH_2]_2—$,

$$—CH_2CH(OZ)CH_2—[OCH_2CH(R_{24})]_m—OCH_2CH—(OZ)CH_2—$$

and

$$—CH_2CH(OZ)CH_2—[OWO—CH_2CH(OZ)CH_2]_n—.$$

While these compounds are prepared by linking two or more piperidine rings with polyglycidyl compounds, the resulting compounds do not contain oxirane rings

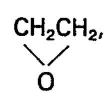

and are not good heat and light stabilizers for polyvinyl chloride resins.

The FR—A—76 33960 describes compounds according to the formula:

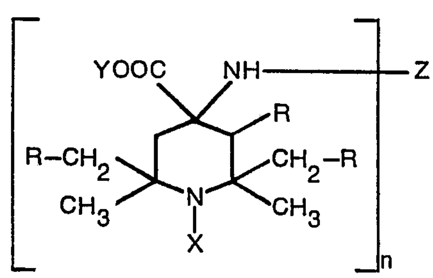

and their addition-salts with acids wherein:

R represents a hydrogen atom or an alkyl group having from 1 to 3 carbon atoms;

X represents a hydrogen atom; an oxygen radical; an alkyl group having from 1 to 18 carbon atoms; an alkenyl group having from 3 to 6 carbon atoms; a 2-propynyl group; an alkoxyalkyl group having from 1 to 3 carbon atoms in the alkyl fragment and from 1 to 18 carbon atoms in the alkoxy fragment; a cyanoalkyl group with 2 or 3 carbon atoms; a 2,3-epoxypropyl group; an aliphatic acyl group having from 1 to 12 carbon atoms; an aralkyl group eventually substituted in its aryl residue; a group of the formula $—CH_2CH(R^1)OR^2$ wherein $R^1$ represents a hydrogen atom, a methyl group or a phenyl group and $R^2$ represents a hydrogen atom, or an aliphatic, aromatic, araliphatic or alicyclic acyl group having up to 18 carbon atoms; a group of formula $—CH_2COOR^3$ wherein $R^3$ represents an alkyl group having from 1 to 18 carbon atoms, an alkenyl group having from 3 to 6 carbon atoms, a phenyl group, an aralkyl group having 7 or 8 carbon atoms, or a cyclohexyl group; or a group of formula $—COOR^4$ wherein $R^4$ represents an alkyl group having from 1 to 8 carbon atoms, a benzyl group, or a phenyl group;

Y represents an alkyl group having from 1 to 18 carbon atoms, an alkenyl group having from 3 to 6 carbon atoms; an aralkyl group eventually substituted in its aryl residue; or an alkoxyalkyl group having from 2 to 4 carbon atoms;

$n = 1, 2$ or 3; and

if $n = 1$:

Z represents a hydrogen atom; an alkyl group having from 1 to 18 carbon atoms; an alkenyl group with 3 or 4 carbon atoms; an alkanoyl group having from 2 to 18 carbon atoms, an alkenoyl group having from 3 to 5 carbon atoms; an aromatic acyl group eventually substituted in its aryl residue; a cinnamoyl group; a 3-(3,5-di-t-butyl-4-hydroxyphenyl)propionyl group; a phenylacetyl group; a phenoxyacetyl group; a cyclohexanecarbonyl group; an aralkyl group eventually substituted in its aryl residue; a group of formula $—CH_2CH_2OR^5$ wherein $R^5$ represents a hydrogen atom, or an aliphatic, aromatic, araliphatic or alicyclic acyl group containing up to 18 carbon atoms; a group of formula $—SO_2R^6$ wherein $R^6$ represents an alkyl group having from 1 to 3 carbon atoms, a phenyl group or a tolyl group; a group of formula $—COOR^7$ wherein $R^7$ represents an alkyl group having from 1 to 8 carbon atoms or a benzyl group; a group of formula $—CONR^8R^9$ wherein $R^8$ represents a hydrogen atom or an alkyl group having from 1 to 4 carbon atoms and $R^9$ represents a hydrogen atom, an alkyl group having from 1 to 18 carbon atoms, a phenyl group eventually substituted, a naphtyl group, a benzyl group or a cyclohexyl group, or $R^8$ and $R^9$ together represent an alkylene group having from 4 to 6 carbon atoms; or a group of formula $—CSNHR^{10}$ wherein $R^{10}$ represents an alkyl group having from 1 to 4 carbon atoms or a phenyl group;

if $n = 2$:

Z represents an alkylene group having from 1 to 10 carbon atoms; a 2-butenylene group; an m- or p-xylylene group; a carbonyl group; a sulfinyl group; a sulfonyl group; an oxalyl group; a group of formula $—CO—R^{11}—CO—$ wherein $R^{11}$ represents an alkylene group having from 1 to 10 carbon atoms which eventually may be interrupted by a sulfur atom; an alkenylene group having from 2 to 4 carbon atoms; an arylene group with 6 or 10 carbon atoms in its aryl residue; or a cyclohexylene group; or a group of formula $—CONH—R^{12}—NHCO—$ wherein $R^{12}$ represents an alkylene group having from 2 to 8 carbon atoms, a phenylene group, a tolylene group, a naphtylene group, a xylylene group, a group of formula

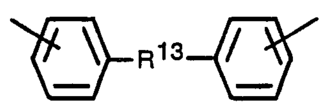

wherein $R^{13}$ represents an oxygen atom or a methylene group, or a group of formula

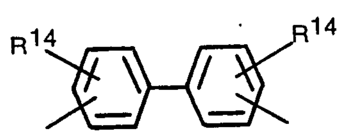

wherein $R^{14}$ represents a hydrogen atom or a methyl group;

if $n = 3$:

Z represents a group of formula

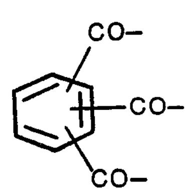

,

a group of formula

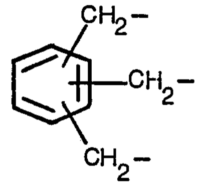

or a group of formula

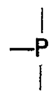 or 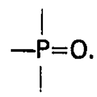

The US—P 4 460 725 describes polyethers containing 2,2,6,6-tetramethyl piperidinyl carboxylic acid ester or ether groups comprising polymeric units having the structure

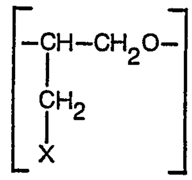

wherein

X is selected from the group consisting of:

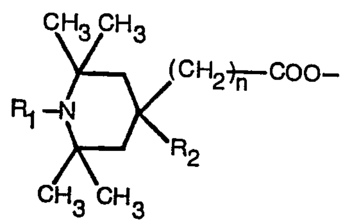

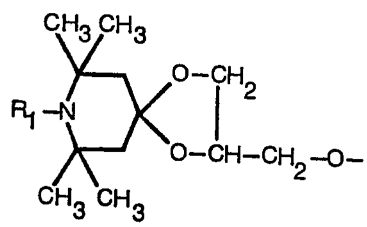

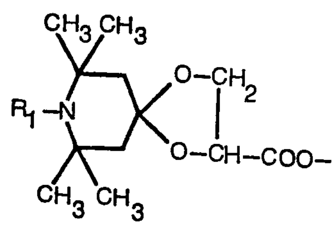

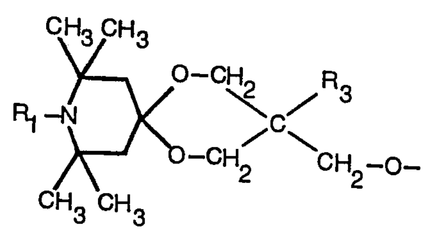

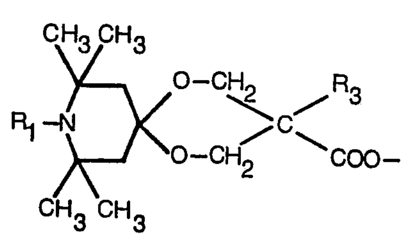

$R_1$ is selected from the group consisting of hydrogen, —O, alkyl, hydroxy alkyl and epoxyalkyl having from 1 to about 18 carbon atoms, acyl having from 1 to about 18 carbon atoms, cycloalkyl having from 3 to about 18 carbon atoms; phenyl; phenalkyl and alkylphenyl having from 7 to about 24 carbon atoms;

$R_2$ is hydrogen or hydroxy;

$n_1$ is 0 or 1;

$R_3$ is lower alkyl having from 1 to 6 carbon atoms; and

n is the average number of such units in the polyether, within the range from 2 to 50.

However also the piperidyl derivatives described in these documents are still no satisfactory heat and light stabilizers for polyvinyl chloride resins.

In accordance with the invention, α-hydroxy-β-2,2,6,6-tetramethyl piperidyl ethylene oxiranes are provided which are effective to improve the resistance to deterioration when exposed to heat and light of polyvinyl chloride resin, and which are the reaction products of a compound having the formula Ia

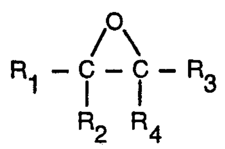

in which at least one of the groups $R_1$, $R_2$, $R_3$ and $R_4$ includes an oxirane group, the total of such oxirane groups in $R_1$, $R_2$, $R_3$ and $R_4$ being within the range of one to twenty and $R_1$, $R_2$, $R_3$ and $R_4$ being selected from the group consisting of hydrogen, aliphatic, aromatic, mixed aromatic aliphatic cycloaliphatic and mixed cycloaliphatic aliphatic hydrocarbon groups having from one to thirty carbon atoms and heterocyclic groups having from one to three ring nitrogen and/or oxygen atoms and five to three ring carbon atoms and a 2,2,6,6-tetramethylpiperidyl compound containing in the molecule the residue of formula Ib

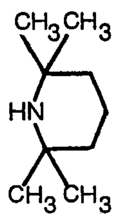

to produce a compound in which said 2,2,6,6-tetramethyl piperidyl residue becomes linked to one carbon of the oxirane ring of the compound of formula Ia and the epoxy oxygen becomes a hydroxyl group attached to the other carbon atoms of the oxirane ring.

Exemplary $R_1$, $R_2$, $R_3$ and $R_4$ alkyl groups include methyl, ethyl, propyl, isopropyl, butyl, isobutyl, secondary butyl, tertiary butyl, amyl, isoamyl, secondary amyl, tertiary amyl, butyl, isobutyl, tertiary butyl, hexyl, isohexyl, tertiary hexyl, heptyl, isoheptyl, octyl, 2-ethylhexyl, isooctyl, tertiary octyl, nonyl, isononyl, tertiary nonyl, decyl, isodecyl, tertiary decyl, undecyl, dodecyl, tridecyl, tetradecyl, hexadecyl, octadecyl, eicosyl and behenyl.

Exemplary $R_1$, $R_2$, $R_3$ and $R_4$ alkenyl and alkadienyl include hexenyl, allyl, octenyl, butenyl, butadienyl and isoprenyl.

Exemplary $R_1$, $R_2$, $R_3$ and $R_4$ cycloalkyl and cycloalkenyl include cyclopropyl, cyclobutyl, cyclopentyl, cyclohexyl, cycloheptyl, cylooctyl and cyclododecyl; cyclopentenyl, cyclohexenyl, cyclopentadienyl and cyclobutadienyl.

Examplary $R_1$, $R_2$, $R_3$ and $R_4$ aryl include phenyl and naphthyl.

Exemplary $R_1$, $R_2$, $R_3$ and $R_4$ alkaryl and arylalkyl include phenethyl, phenmethyl, phenpropyl, phenbutyl, xylyl, tolyl and mesityl.

Exemplary $R_1$, $R_2$, $R_3$ and $R_4$ heterocyclic include isocyanurate and triazine.

Any or all of $R_1$, $R_2$, $R_3$ and $R_4$ can contain one or more oxirane rings in the molecule. Exemplary polyepoxy compounds containing such groups of Formula Ia include polyglycidylethers or poly-β-methylglycidylethers prepared from phenol compounds, such as Bisphenol A, bis(hydroxyphenyl)methane, resorcinol, tetra(hydroxyphenyl) methane and phenolic novolak reacted with epichlorohydrin or β-methylepichlorohydrin; polyglycidylethers or poly-β-methylglycidylethers prepared from polyols such as ethylene glycol, polyethylene glycol, 2,2-bis(4-hydroxycyclohexyl) propane, glycerol and 1,1,1-trimethylolpropane reacted with epichlorohydrin or β-methylepichlorohydrin; polyglycidylesters or poly(β-methyl)glycidylesters prepared from polycarboxylic acids such as adipic acid, sebacic acid, phthalic acid, tetrahydrophthalic aciod and hexahydrophthalic acid reacted with epichlorohydrin or β-methylepichlorohydrin; epoxidized polybutadiene; tri(2,3-epoxypropyl) isocyanurate; epoxidized natural oils such as epoxidized soybean oil, epoxidized linseed oil, epoxidized fish oil and epoxidized tallow; and cycloaliphatic polyepoxy compounds such as vinylcyclohexenediepoxide, dicyclopentadienediepoxid, and 3,4-epoxycyclohexylmethyl-3,4-epoxycyclohexanecarboxylate.

Exemplary 2,2,6,6-tetramethyl piperidine compounds which can be reacted with these polyepoxides include esters, amides or imides of 2,2,6,6-tetramethyl-4-piperidinol, 9-aza-8,8,10,10-tetramethyl-3-ethyl 1,5-dioxaspiro[5.5]-3-undecylmethanol or 2,2,6,6-tetramethyl-4-(alkyl)-piperidines with organic carboxylic acids or oxy acids; ketals of 2,2,6,6-tetramethyl-4-piperidone with (poly)alcohols; and 7,7,9,9-tetramethyl-1,3,8-triazaspiro [4.5] decanes.

Preferred 2,2,6,6-tetramethyl piperidine compounds include:

1. 4-Benzoyloxy-2,2,6,6-tetramethyl piperidine
2. 9-Aza-8,8,10,10-tetramethyl-3-ethyl-1,5-dioxaspiro[5.5]-3-undecylmethyllaurate
3. 4-Stearoyloxy-2,2,6,6-tetramethyl piperidine
4. 4(3,5-Di-t-butyl-4-hydroxyphenylpropionyloxy)-2,2,6,6-tetramethyl piperidine
5. 4-(3,5-Di-t-butyl-4-hydroxybenzoyloxy)-2,2,6,6-tetramethyl piperidine
6. Bis(2,2,6,6-tetramethyl-4-piperidyl)sebacate
7. Bis(2,2,6,6-tetramethyl-4-piperidyl)dodecanedioate
8. Bis(2,2,6,6-tetramethyl-4-piperidyl)terephthalate
9. Bis(9-aza-8,8,10,10-tetramethyl-3-ethyl-1,5-dioxaspiro[5.5]-3-undecylmethyl)methyliminodiacetate
10. Tris(2,2,6,6-tetramethyl-4-piperidyl)citrate
11. Tris(2,2,6,6-tetramethyl-4-piperidyl)nitrilotriacetate
12. Tris(2,2,6,6-tetramethyl-4-piperidyl)butanetricarboxylate
13. Tris(2,2,6,6-tetramethyl-4-piperidyl)trimellitate
14. Bis(2,2,6,6-tetramethyl-4-piperidyl)-mono(tridecyl)trimellitate
15. Tetra(2,2,6,6-tetramethyl-4-piperidyl)pyromellitate
16. Tetra(2,2,6,6-tetramethyl-4-piperidyl)-1,3-bis(aminomethyl)-cyclohexanetetraacetate
17. Tetra(2,2,6,6-tetramethyl 4-piperidyl)-1,2,3,4-butanetetracarboxylate
18. Tris(2,2,6,6-tetramethyl-4-piperidyl)-mono(isotridecyl)-1,2,3,4-butanetetracarboxylate
19. Bis(2,2,6,6-tetramethyl-4-piperidyl)-di(isotridecyl)-1,2,3,4-butanetetracarboxylate
20. N,N'-Bis(2,2,6,6-tetramethyl-4 piperidyl)oxalamide
21. N-(2,2,6,6-Tetramethyl-4-piperidyl)dodecylsuccinimide
22. 3,9-Bis(1,1-dimethyl 2-(tris(2,2,6,6-tetramethyl-4-piperidyloxycarbonyl)butylcarbonyloxy)ethyl)-2,4,8,10-tetraoxaspiro[5.5] undecane
23. 2,4,6-Tris(2,2,6,6-tetramethyl-4-piperidyloxy)-s-triazine
24. 2,4,6-Tris(2,2,6,6-tetramethyl-4-piperidylamino)-s-triazine
25. 2-Dibutylamino-4,6-bis(9-aza-8,8,10,10-tetramethyl-3-ethyl 1,5-dioxaspiro[5.5]-3-undecylmethoxy)-s-triazine
26. N,N'-Bis(4,6-bis(9-aza-8,8,10,10-tetramethyl-3-ethyl 1,5-dioxaspiro[5.5]-3-undecylmethoxy)-s-triazine-2-yl)piperazine
27. Bis(9-aza-8,8,10,10-tetramethyl-3-ethyl-1,5-dioxaspiro[5.5] 3-undecylmethyl)carbonate
28. Bis[9-aza-8,8,10,10-tetramethyl 3-ethyl-1,5-dioxaspiro[5.5] 3-undecylmethyl)-hydrogenated Bisphenol A-dicarbonate
29. Tris(2,2,6,6-tetramethyl-4-piperidyl)phosphite
31. Bis(9-aza-8,8,10,10-tetramethyl-3-ethyl-1,5-dioxaspiro[5.5]-3-undecylmethyl)-pentaerythritoldiphosphite
pentaerythritoldiphosphite
32. Tetra(2,2,6,6-tetramethyl-4-piperidyl)-Bisphenol A-diphosphite
33. Tris(2,2,6,6-tetramethyl-4-piperidyl)phosphate
34. 3,5-Di-t-butyl-4-hydroxybenzyl-bis(2,2,6,6-tetramethyl 4-piperidyl) phosphonate
35. Phenyl-bis(2,2,6,6-tetramethyl 4-piperidyl) phosphinate
36. Bis(9-aza-8,8,10,10-tetramethyl-3-hydroxymethyl-1,5-dioxaspiro[5.5]-3 undecylmethyl) ether
37. Bis(9 axa 8,8,10,10-tetramethyl-3 octanoyloxymethyl 1,5-dioxaspiro[5.5] 3-undecylmethyl) ether
38. 3-Octyl 7,7,9,9-tetramethyl-1,3,8-triazaspiro [4.5] decane-2,4-dione

Reaction of the polyepoxide with the 2,2,6,6-tetramethyl piperidyl compound proceeds rapidly at elevated temperatures within the range from about 80 to 250°C, preferably from about 120 to about 230°C. The reaction can be carried out in the presence of an inert organic solvent, but if the reactants are liquids, a solvent is unnecessary. The proportion of piperidine compound to polyepoxide is within the range from 0.01 to 0.5, preferably from 0.03 to 0.3, moles of piperidyl group per mole of reacted oxirane group in the polyepoxide.

The following Examples illustrate preparation of these compounds.

## Example I

Bisphenol A diglycidylether, 50 grams, and tetra(2,2,6,6-tetramethyl-4-piperidyl)-1,2,3,4-butanetetracarboxylate, 10 grams, were stirred at 200°C for one hour under a stream of nitrogen. Analysis using high speed liquid chromatography showed that all of the piperidine compound had been consumed at the end of this time. The reaction product was a pale yellow viscous liquid, 60 grams. The molar ratio of oxirane groups to piperidine groups was 1:0.172.

Example II

Tris(2,3-epoxypropyl)isocyanurate, 50 grams, was dissolved in 50 grams of pseudocumene and 25 grams of tetra(2,2,6,6-tetramethyl-4-piperidyl-1,2,3,4-butanetetracarboxylate added. The solution was heated and stirred for two hours under a stream of nitrogen at reflux temperature. Analysis using high speed liquid chromatography showed that all of the piperidine compound had been consumed at the end of this time. The solvent was distilled off, obtaining 70 grams of a pale yellow glassy solid. The molar ratio oxirane groups to piperidine groups was 1:0.084.

Using the above procedure, the following α-hydroxy-β-2,2,6,6-tetramethyl piperidyl ethylene oxiranes were prepared.

| Example No. | Polyepoxy compound | Piperidine compound | Molar ratio Oxirane: Piperidine |
|---|---|---|---|
| III | Bisphenol A diglycidyl-ether (20 g) | Tetra(2,2,6,6-tetramethyl 4-piperidyl)-1,2,3,4-butanetetracarboxylate (1 g) | 1:0.043 |
| IV | Bisphenol A diglycidylether (20 g) | Tetra(2,2,6,6-tetramethyl 4-piperidyl)-1,2,3,4-butanetetracarboxylate (2 g) | 1:0.086 |
| V | Bisphenol A diglycidylether (20 g) | Tetra(2,2,6,6-tetramethyl 4-piperidyl)-1,2,3,4-butanetetracarboxylate (3 g) | 1:0.129 |
| VI | Tris(2,3-epoxypropyl)isocyanurate (50 g) | Tetra(2,2,6,6-tetramethyl 4-piperidyl)-1,2,3,4-butanetetracarboxylate (20 g) | 1:0.201 |
| VII | 3,4-Epoxycyclohexylmethyl-3,4-epoxycyclohexanecarboxylate (30 g) | Tetra(2,2,6,6-tetramethyl 4-piperidyl)-1,2,3,4-butanetetracarboxylate (4 g) | 1:0.085 |
| VIII | 3,4-Epoxycyclohexylmethyl-3,4-epoxycylohexanecarboxylate (30 g) | Tetra(2,2,6,6-tetramethyl 4-piperidyl)-1,2,3,4-butanetetracarboxylate (3 g) | 1:0.064 |
| IX | Epoxidized-1,2-polybutadiene (Adeka Argus Chemical Co., Ltd. BF-1000; M.W.=1000, oxirane oxygen=7.0%) (10 g) | Tetra(2,2,6,6-tetramethyl 4-piperidyl)-1,2,3,4-butanetetracarboxylate (4 g) | 1:0.415 |
| X | 3,4-Epoxycyclohexylmethyl 3,4-epoxycyclohexanecarboxylate (10 g) | Tetra(2,2,6,6-tetramethyl 4-piperidyl)-1,2,3,4-butanetetracarboxylate (2 g) | 1:0.208 |
| XI | Epoxidized-1,2-polybutadiene (Adeka Argus Chemical Co., Ltd. BF-1000; M.W.=1000, oxirane oxygen=7.0%) (20 g) | Tetra(2,2,6,6-tetramethyl 4-piperidyl)-1,2,3,4-butanetetracarboxylate (1 g) | 1:0.058 |
| XII | Epoxidized-1,2-polybutadiene (Adeka Argus Chemical Co., Ltd. BF-1000; M. W.=1000, oxirane oxygen=7.0%) (20 g) | Tetra(2,2,6,6-tetramethyl 4-piperidyl)-1,2,3,4-butanetetracarboxylate (2 g) | 1:0.0116 |
| XIII | Bispenol A diglycidylether (30 g) | Bis(2,2,6,6-tetramethyl-4-piperidyl)-di(tridecyl)-1,2,3,4-butanetetracarboxylate (3 g) | 1:0.039 |
| XIV | Bisphenol A diglycidylether (30 g) | Bis(2,2,6,6-tetramethyl-4-piperidyl)-di(tridecyl)-1,2,3,4-butanetetracarboxylate (4 g) | 1:0.052 |
| XV | Tris(2,3-epoxypropyl)isocyanurate (50 g) | Bis(2,2,6,6-tetramethyl-4-piperidyl)-di(tridecyl)-1,2,3,4-butanetetracarboxylate (4 g) | 1:0.018 |
| XVI | Tris(2,3-epoxypropyl)isocyanurate (50 g) | Bis(2,2,6,6-tetramethyl-4-piperidyl)-di(tridecyl)-1,2,3,4-butanetetracarboxylate (5 g) | 1:0.023 |
| XVII | 3,4-Epoxycyclohexylmethyl 3,4-epoxycyclohexanecarboxylate (10 g) | Bis(2,2,6,6-tetramethyl-4-piperidyl)-di(tridecyl)-1,2,3,4-butanetetracarboxylate (2 g) | 1:0.058 |

| Example No. | Polyepoxy compound | Piperidine compound | Molar ratio Oxirane: Piperidine |
|---|---|---|---|
| XVIII | Epoxidized-1,2-polybutadiene (Adeka Argus Chemical Co., Ltd. BF-1000; M.W.=1000, oxirane oxygen=7.0%) (10 g) | Bis(2,2,6,6-tetramethyl-4-piperidyl)-di(tridecyl)-1,2,3,4-butanetetracarboxylate (4 g) | 1:0.187 |
| XIX | Bisphenol A diglycidyl ether (30 g) | 3,9-Bis(1,1-dimethyl-2-(tris(2,2,6,6-tetramethyl-4-piperidyloxycarbonyl)butylcarbonyloxy)-ethyl)2,4,8,10-tetraoxaspiro[5.5]undecane (3 g) | 1:0.065 |
| XX | Bisphenol A diglycidyl ether (10 g) | 3,9-Bis(1,1-dimethyl-2-(tris(2,2,6,6-tetramethyl-4-piperidyloxycarbonyl)butylcarbonyloxy)-ethyl)2,4,8,10-tetraoxaspiro[5.5]undecane (4 g) | 1:0.087 |
| XXI | Tris(2,3-epoxypropyl) isocyanurate (20 g) | 3,9-Bis(1,1-dimethyl-2-(tris(2,2,6,6-tetramethyl-4-piperidyloxycarbonyl)butylcarbonyloxy)-ethyl)2,4,8,10-tetraoxaspiro[5.5]undecane (20 g) | 1:0.378 |
| XXII | Bisphenol A diglycidylether (30 g) | Bis(2,2,6,6-tetramethyl-4 piperidyl)sebacate (1.5 g) | 1:0.035 |
| XXIII | Bisphenol A diglycidylether (30 g) | Bis(2,2,6,6-tetramethyl-4-piperidyl)sebacate (2 g) | 1:0.047 |
| XXIV | Tris(2,3-epoxypropyl) isocyanurate (20 g) | Bis(2,2,6,6-tetramethyl-4-piperidyl)sebacate (10 g) | 1:0.206 |
| XXV | Bisphenol A diglycidylether (30 g) | N-(2,2,6,6-tetramethyl-4-piperidyl)dodecylsuccinimide (2 g) | 1:0.028 |
| XXVI | Bisphenol A diglycidylether (30 g) | N-(2,2,6,6-tetramethyl-4-piperidyl)dodecylsuccinimide (3 g) | 1:0.042 |

The amount of α-hydroxy-β-2,2,6,6-tetramethyl piperidyl ethylene oxirane employed with polyvinyl chloride resin is sufficient to improve the resistance to deterioration when exposed to heat and light of the polymer, and is within the range from about 0.01 to about 10 parts by weight, preferably within the range from about 0.1 to about 5 parts by weight, per 100 parts by weight of polyvinyl chloride resin.

The stabilizer compositions of the invention can be used as stabilizers with any polyvinyl chloride resin. The term "polyvinyl chloride" as used herein is inclusive of any polymer formed at least in part of the recurring group:

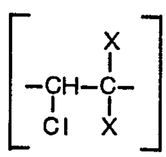

and having a chlorine content in excess of 49%. In this group, the X groups can each be either hydrogen or chlorine. In polyvinyl chloride homopolymers, each of the X groups is hydrogen. Thus, the term includes not only polyvinyl chloride homopolymers but also after-chlorinated polyvinyl chloride, such as those disclosed in British patent No. 893,288, and also copolymers of vinyl chloride in a major proportion and other copolymerizable monomers in a minor proportion, such as copolymers of vinyl chloride and vinyl acetate, copolymers of vinyl chloride with maleic or fumaric acids or esters, and copolymers of vinyl chloride with styrene, propylene, and ethylene. The invention also is applicable to mixtures of polyvinyl chloride in a major proportion with other synthetic resins such as chlorinated polyethylene or a copolymer of acrylonitrile, butadiene and styrene. Among the polyvinyl chlorides which can be stabilized are the uniaxially-stretch oriented polyvinyl chlorides described in U.S. patent No. 2,934,593 to Isaksem et al, that is, syndiotactic polyvinyl chloride, as well as atactic and isotactic polyvinyl chlorides.

The α-hydroxy-β-2,2,6,6-tetramethyl piperidyl ethylene oxiranes of the invention both with and without supplementary heat and light stabilizers are excellent stabilizers for both unplasticized or rigid polyvinyl chloride resins, as well as plasticized polyvinyl chloride resins. The rigid polyvinyl chloride resins are defined as those containing at most 10% plasticizer. Plasticized resins contain at least 10% plasticizer, and can contain as much as 50 to 60% plasticizers according to the degree of plasticization desired. When plasticizers are to be employed, they may be incorporated into the polyvinyl chloride resin using

conventional means. Conventional plasticizers can be used, such as dioctyl phthalate, dioctyl sebacate and tricresyl phosphate.

Particularly useful plasticizers are the epoxy higher fatty acid esters having from about twenty to about one hundred fifty carbon atoms. Such esters will initially have had unsaturation in the alcohol or acid portion of the molecule, which is taken up by the formation of the epoxy group.

Typical unsaturated acids are oleic, linoleic, linolenic, erucic, ricinoleic and brassidic acids, and these may be esterified with organic monohydride or polyhydric alcohols, the total number of carbon atoms of the acid and the alcohol being within the range stated. Typical monohydric alcohols include butyl alcohol, 2-ethylhexyl alcohol, lauryl alcohol, isooctyl alcohol, stearyl alcohol and oleyl alcohol. The octyl alcohols are preferred. Typical polyhydric alcohols include pentaerythritol, glycerol, ethylene glycol, 1,2-propylene glycol, 1,4-butylene glycol, neopentyl glycol, ricinoleyl alcohol, erythritol, mannitol and sorbitol. Glycerol is preferred. These alcohols may be fully or partially esterified with the epoxidized acid. Also useful are the epoxidized mixtures of higher fatty acid esters found in naturally occurring oils such as epoxidized soybean oil, epoxidized olive oil, epoxidized cottonseed oil, epoxidized tall oil fatty acid esters, epoxidized linseed oil and epoxidized tallow. Of these, epoxidized soybean oil is preferred.

The alcohol can contain the epoxy group and have a long or short chain, and the acid can have a short or long chain, such as epoxy stearyl acetate, epoxy stearyl stearate, glycidyl stearate, and polymerized glycidyl methacrylate.

A small amount, usually not more than 1.5%, of a parting agent or lubricant can also be included. Typical parting agents are the higher aliphatic acids, and alkali and alkaline earth metal salts of such acids, having twelve to twenty four carbon atoms, such as stearic acid, lauric acid, palmitic acid and myristic acid; for example, lithium stearate, calcium stearate and calcium palmitate; mineral lubricating oils, polyvinyl stearate, polyethylene and paraffin wax.

Impact modifiers, for improving the toughness or impact-resistance of unplasticized resins, can also be added to the resin composition stabilized by the present invention in minor amounts of usually not more than 10%. Examples of such impact modifiers include chlorinated polyethylene, ABS polymers, and polyacrylate butadiene graft copolymers.

The α-hydroxy-β-2,2,6,6-tetramethyl piperidyl ethylene oxiranes of the invention can be combined with conventional heat stabilizers, such as phenolic antioxidants, polyvalent metal salts of organic acids, organic phosphites, thioethers, organotin compounds, and other known heat stabilizers, thereby constituting light and heat stabilizer compositions of the invention.

The phenolic antioxidant contains one or more phenolic hydroxyl groups, and one or more phenolic nuclei, and can contain from about eight to about three hundred carbon atoms. In addition, the phenolic nucleus can contain an oxy or thio ether group.

The alkyl substituted phenols and polynuclear phenols, because of their molecular weight, have a higher boiling point, and therefore are preferred because of their lower volatility. There can be one or a plurality of alkyl groups of one or more carbon atoms. The alkyl group or groups including any alkylene groups between phenol nuclei preferably aggregate at least four carbon atoms. The longer the alkyl or alkylene chain, the better the compatibility with polypropylene, inasmuch as the phenolic compound then acquires more of an aliphatic hydrocarbon character, and therefore there is no upper limit on the number of alkyl carbon atoms. Usually, from the standpoint of availability, the compound will not have more than about eighteen carbon atoms in an alkyl, alicyclidene and alkylene group, and a total of not over about fifty carbon atoms. The compounds may have from one to four alkyl radicals per phenol nucleus.

The phenol contains at least one and preferably at least two phenolic hydroxyls, the two or more hydroxyls being in the same ring, if there is only one. In the case of bicyclic phenols, the rings can be linked by thio or oxyether groups, or by alkylene, alicyclidene or arylidene groups.

The monocyclic phenols which can be employed have the structure:

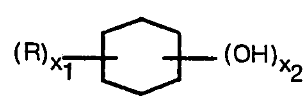

R is selected from the group consisting of hydrogen; halogen; and organic radicals containing from one to about thirty carbon atoms, such as alkyl, aryl, alkenyl, alkaryl, aralkyl, cycloalkenyl, cycloalkyl, alkoxy and acyl

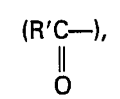

where R′ is aryl, alkyl or cycloalkyl.

$x_1$ and $x_2$ are integers from one to four, and the sum of $x_1$ and $x_2$ does not exceed six.

The polycyclic phenol phenol is one having at least two aromatic nuclei linked by a polyvalent linking radical, as defined by the formula:

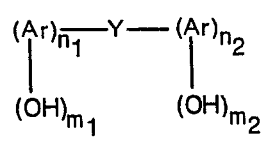

wherein

Y is a polyvalent linking group selected from the group consisting of oxygen; carbonyl; sulfur; sulfinyl; aromatic, aliphatic and cycloaliphatic hydrocarbon groups; and oxyhydrocarbon, thiohydrocarbon and heterocyclic groups. The linking group can have from one to twenty carbon atoms.

Ar is a phenolic nucleus which can be a phenyl or a polycarbocyclic group having condensed or separate phenyl rings; each Ar group contains at least one free phenolic hydroxyl group up to a total of five. The Ar rings can also include additional rings connected by additional linking nuclei of the type Y, for example, Ar—Y—Ar—Y—Ar.

$m_1$ and $m_2$ are numbers from one to five, and $n_1$ and $n_2$ are numbers of one or greater, and preferably from one to four.

The aromatic nucleus Ar can, in addition to phenolic hydroxyl groups, include one or more inert substituents. Examples of such inert substituents include hydrogen, halogen atoms, e.g., chlorine, bromine and fluorine; organic radicals containing from one to about thirty carbon atoms, such as alkyl, aryl, alkaryl, aralkyl, cycloalkenyl, cycloalkyl, alkoxy, aryloxy and acyloxy

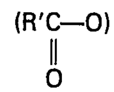

where R' is aryl, alkyl or cyclolalkyl, or thiohydrocarbon groups having from one to about thirty carbon atoms, and carboxyl

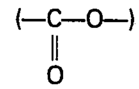

groups. Usually, however, each aromatic nucleus will not have more than about eighteen carbon atoms in any hydrocarbon substituent group. The Ar group can have from one to four substituent groups per nucleus.

Typical aromatic nuclei include phenyl, naphthyl, phenanthryl, triphenylenyl, anthracenyl, pyrenyl, chrysenyl, and fluoroenyl groups.

When Ar is a benzene nucleus, the polyhydric polycyclic phenol has the structure:

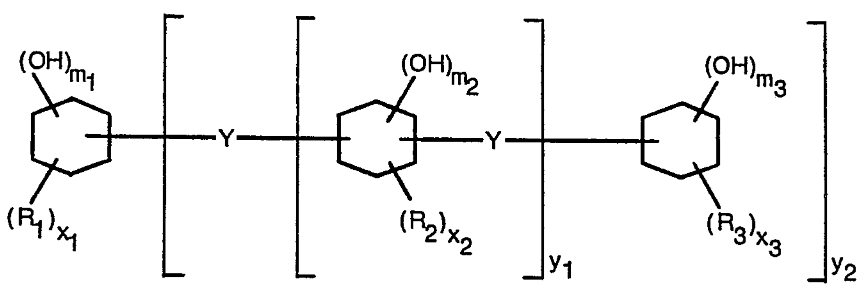

wherein

$R_1$, $R_2$ and $R_3$ are inert substituent groups as described in the previous paragraph;
$m_1$ and $m_3$ are integers from one to a maximum of five;
$m_2$ is an integer from one to a maximum of four;
$x_1$ and $x_3$ are integers from zero to four, and
$x_2$ is an integer from zero to three;
$y_1$ is an integer from zero to about six and
$y_2$ is an integer from one to five, preferably one or two.

Preferably, the hydroxyl groups are located ortho and/or para to Y.

Exemplary Y groups are alkylene, alkylidene, and alkenylene; arylene, alkyl arylene, arylalkylene; cycloalkylene, cycloalkylidene; and oxa- and thia-substituted such groups; tetrahydrofuranes, esters and triazino groups. The Y groups are usually bi, tri, or tetravalent, connecting two, three or four Ar groups. However, higher valency Y groups connecting more than four Ar groups, can also be used. According to their constitution, the Y groups can be assigned to subgenera as follows:

1) Y groups where at least one carbon in a chain or cyclic arrangement connect the aromatic groups, such as:

$-CH_2-CH_2-$;$-(CH_2)_5-$; $-CH_2-$; 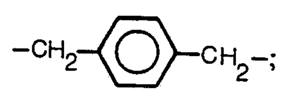 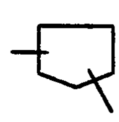 ; 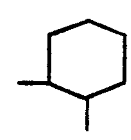 ;

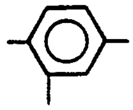 ; 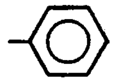 ; 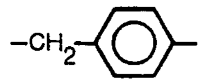 ; 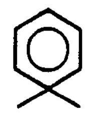 ; 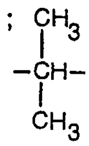 ; 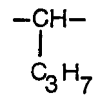 ;

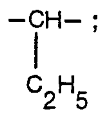 ; 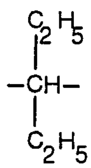 ; 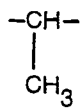 ; 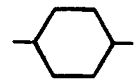 ; 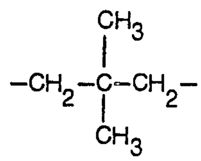 ;

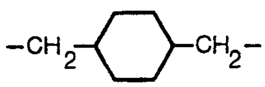 ; 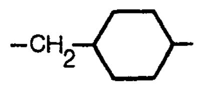 ; 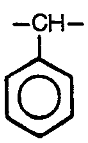 ; 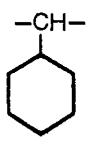 ;

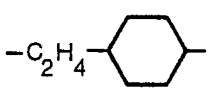 ; 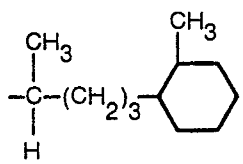 ; 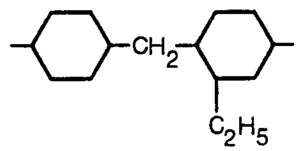

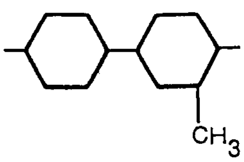 ; 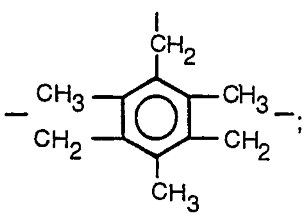 ; 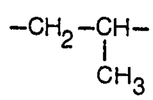 ; 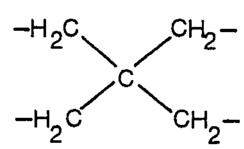 ;

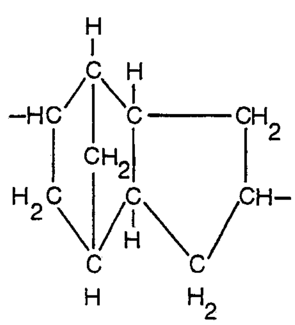 ; 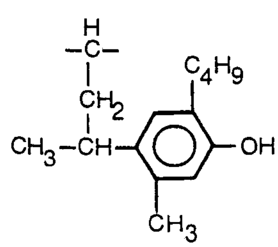 ;

2) Y groups where only atoms other than carbon link the aromatic rings, such as

—O—, —S—, 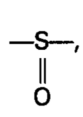, 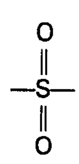 and $—(S)_x$

where x is a number from one to ten;

3) Y groups made up of more than a single atom including both carbon and other atoms linking the aromatic nuclei, such as:

$-CH_2-O-CH_2-$ ; 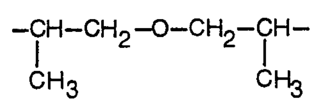 ; $-O-CH_2-CH_2-O-$ ;

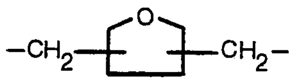 ; 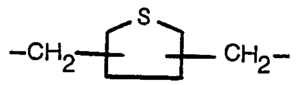 ; $-S-CH_2-S-$ ;

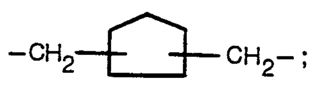 ; 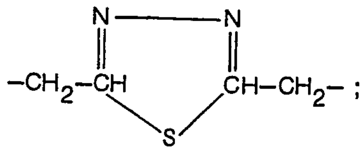 ; 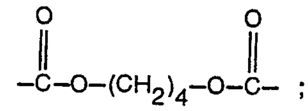 ;

$C[-CH_2OOCCH_2CH_2-]_4\equiv$ ; 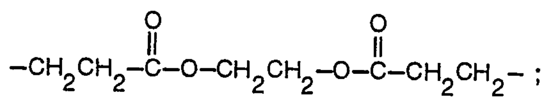 ;

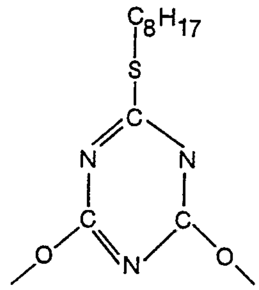 ; $-CH_2-S-$ ; $-CH_2-S-CH_2-$ ; and 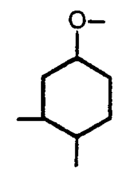

Although the relation of effectiveness to chemical structure is insufficiently understood, many of the most effective phenols have Y groups of subgenus 1), and accordingly this is preferred. Some of these phenols can be prepared by the alkylation of phenols or alkyl phenols with polyunsaturated hydrocarbons such as dicyclopentadiene or butadiene.

Representative phenols include guaiacol, resorcinol monoacetate, vanillin, butyl salicylate, 2,6-di-tert-butyl-4-methyl phenol, 2-tert-butyl-4-methoxy phenol, 2,4-dinonyl phenol, 2,3,4,5-tetradecyl phenol, tetrahydro-α-naphthol, o-, m- and p-cresol, o-, m- and p-phenylphenol, o-, m- and p-xylenols, the carvenols, symmetrical xylenol, thymol, o-, m- and p-nonylphenol, o-, m- and p-dodecyl-phenol, and o-, m- and p-octyl-phenol, o- and m-tert-butyl-p-hydroxy-anisole, p-n-decyloxy-phenol, p-n-decyloxy-cresol, nonyl-n-decyloxycresol, eugenol, isoeugenol, glyceryl monosalicylate, methyl-p-hydroxy-cinnamate, 4-benzyloxy-phenol, p-acetylaminophenol, p-stearyl-aminophenol, methyl-p-hydroxybenzoate, p-dichlorobenzoyl-aminophenol, p-hydroxysalicyl anilide, stearyl-(3,5-di-methyl-4-hydroxy-benzyl)thioglycolate, stearyl-β-(4-hydroxy-3,5-di-t-butylphenyl)propionate, distearyl-3,5-di-t-butyl-4-hydroxybenzylphosphonate, and distearyl (4-hydroxy-3-methyl-5-t-butyl)benzylmalonate.

Exemplary polyhydric phenols are orcinol, propyl gallate, catechol, resorcinol, 4-octyl-resorcinol, 4-dodecyl-resorcinol, 4-octadecyl-catechol, 4-isooctyl-phloroglucinol, pyrogallol, hexahydroxybenzene, 4-isohexylcatechol, 2,6-di-tertiary-butyl-resorcinol, 2,6-di-isopropyl-phloroglucinol.

Exemplary polyhydric polycyclic phenols are methylene bis-(2,6-di-tertiary-butyl-phenol), 2,2-bis-(4-hydroxyphenyl)propane, methylene-bis-(p-cresol), 4,4'-benzylidene bis(2-tertiary-butyl-5-methyl-phenol), 4,4'-cyclo-hexylidene bis-(2-tertiary-butylphenol), 2,2'-methylene-bis-(4-methyl-6-(1'-methyl-cyclohexyl)-phenol, 2,6-bis-(2'-hydroxy-3'-tertiary-butyl-5'-methylbenzyl)-4-methylphenol, 4,4'-bis-(2-tertiary-butyl-5-methyl-phenol), 2,2'-bis-(4-hydroxy-phenyl)butane, ethylene bis-(p-cresol), 4,4'-oxobis-phenol, 4,4'-oxobis-(3-methyl-5-isopropyl-phenol), 4,4'-oxobis-(3-methyl-phenol), 2,2'-oxobis-(4-dodecyl-phenol), 2,2'-oxobis-(4-methyl-5-tertiary-butyl-phenol), 4,4'-thio-bis-phenol; 4,4'-thio-bis-(3-methyl-6-tertiary-butyl-phenol), 2,2'-thio-bis-(4-methyl-6-tertiary-butyl-phenol), 4,4'-n-butylidene-(2-t-butyl-5-methylphenol), 2,2'-methylene-bis-(4-methyl-6-(1'-methyl-cyclohexyl)phenol), 4,4'-cyclohexylene bis-(2-tertiary-butyl-phenol), 2,6-bis-(2'-hydroxy-3'-t-butyl-5'-methyl-benzyl)-4-methyl-phenol, 4,4'-oxobis(naphthalene-1,5-diol), 1,3'-bis-(naphthalene-2,5-diol)propane, and 2,2'-butylene bis-(naphthalene-2,7-diol), (3-methyl-5-tert-butyl-4-hydroxyphenyl)-4'-hydroxy-phenyl)propane, 2,2'-methylene-bis-(4-methyl-5-isopropylphenol), 2,2'-methylene-bis-(4-methyl-5-isopropylphenol), 2,2'-methylene-bis-(5-tert-butyl-4-chlorophenol), (3,5-di-tert-butyl-4-hydroxyphenyl)-(4'-hydroxyphenyl)ethane,(2-hydroxy-phenyl)-(3',5'-di-tert-butyl-4',4-hydroxy-phenyl) ethane, 2,2'-methylene-bis-(4-octylphenol), 4,4'-propylene-bis-(2-tert-butyl-phenol), 2,2'-isobutylene-bis-(4-nonylphenol), 2,4-bis-(4-hydroxy-3-t-butyl-phenoxy)-6-(n-octylthio)-1,3,5-triazine, 2,4,6-tris-(4-hydroxy-3-t-butyl-phenoxy)-1,3,5-triazine, 2,2'-bis-(3-t-butyl-4-hydroxyphenyl)thiazolo-(5,4-d)thiazole, 2,2'-bis-(3-methyl-5-t-butyl-4-hydroxyphenyl)thiazolo-(5,4-d)-thiazole, 4,4'-bis-(4-hydroxyphenyl) pentanoic acid octadecyl ester, cyclopentylene-4,4'-bis-phenol, 2-ethylbutylene-4,4'-bisphenol, 4,4'-cyclo-octylene-bis-(2-cyclohexylphenol), β,β-thiodiethanol-bis-(3-tert-butyl-4-hydroxyphenoxy acetate), 1,4-butanedio-bis-(3-tert-butyl-4-hydroxyphenoxy acetate), pentaerythritol tetra-(4-hydroxyphenol propionate), 2,4,4'-tri-hydroxy benzophenone, bis-(2-tert-butyl-3-hydroxy-5-methylphenyl)sulfide, bis-(2-tert-butyl-4-hydroxy-5-methylphenyl) sulfide, bis-(2-tert-butyl-4-hydroxy-5-methylphenyl) sulfoxide, bis-(3-ethyl-5-tert-butyl-4-hydroxybenzyl) sulfide, bis-(2-hydroxy-4-methyl-6-tert-butyl-phenyl) sulfide, 4,4'-bis-(4-hydroxyphenol)pentanoic acid octadeyl thiopropionate ester, 1,1,3-tris-(2'-methyl-4-hydroxy-5'-tert-butylphenyl)butane, 1,1,3-tris-(1-methyl-3-hydroxy-4-tert-butylphenyl)butane, 1,8-bis-(2-hydroxy-5-methylbenzoyl-n-octane, 2,2'-ethylene-bis-[4'-(3-tert-butyl-4-hydroxyphenyl)-thiazole], 1-methyl-3-(3-methyl-5-tert-butyl-4-hydroxybenzyl)-naphthalene, 2,2'-(2-butene)-bis-(4-methoxy-6-tert-butylphenol)-bis-[3,3-bis-(4-hydroxy-3-t-butylphenyl)butyric acid] glycol ester, 4,4'-butylidene-bis-(6-t-butyl-m-cresol), 1,1,3-tris-(2-methyl-4-hydroxy-5-t-butylphenyl)butane, 1,3,5-tris-(3,5-di-t-butyl-4-hydroxybenzyl)-2,4,6-trimethylbenzene, tetrakis[methylene-3,3,5-di-t-butyl-4-hydroxyphenyl)propionate], methane, 1,3,5-tris-(3,5-di-t-butyl-4-hydroxybenzyl)isocyanurate, 1,3,5-tris-(3,5-di-t-butyl-4-hydroxyphenyl) propionyl-oxyethyl isocyanurate, 2-octylthio-4,6-di-(4-hydroxy-3,5-di-t-butyl)phenoxy-1,3,5-triazine, 4,4'-thiobis-(6-t-butyl-m-cresol) and penta-erythritol hydroxyphenyl propionate.

A particularly desirable class of polyhydric polycyclic phenols are the dicyclopentadiene polyphenols, which are of the type:

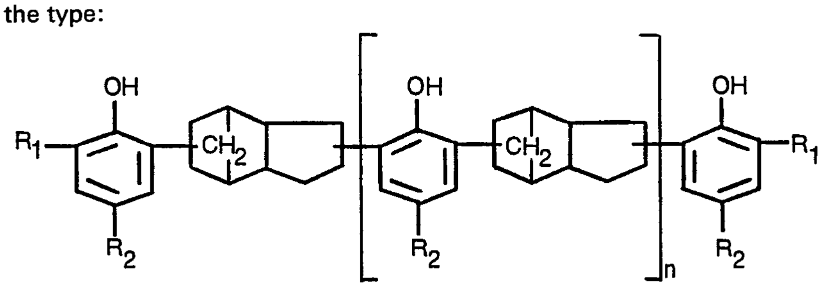

in which

$R_1$ and $R_2$ are lower alkyl, and can be the same or different, and

n is the number of the groups enclosed by the brackets, and is usually from 1 to about 5. These are described in U.S. patent No. 3,567,683, dated March 2, 1971 to Spacht. A commercially available member of this class is Wingstay L, exemplified by dicyclopentadiene tri-(2-tert-butyl-4-methyl-phenol) of the formula:

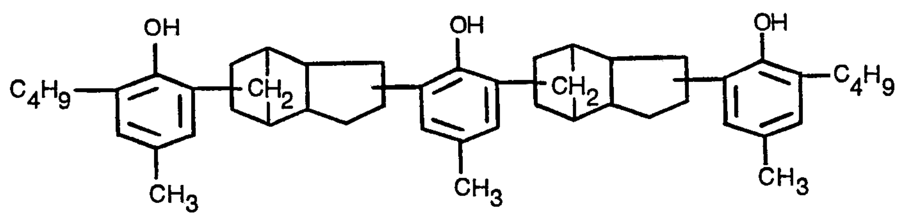

The polyhydric polycyclic phenols used in the invention can also be condensation products of phenols or alkylphenols with hydrocarbons having a bicyclic ring structure and a double bond or two or more double bonds, such as α-pinene, β-pinene, dipentene, limonene, vinylcyclohexene, dicyclopentadiene, allo-ocimene, isoprene and butadiene. These condensation products are usually obtained under acidic conditions in the form of more or less complex mixtures of monomeric and polymeric compounds. However, it is usually not necessary to isolate the individual constituents. The entire reaction product, merely freed from the acidic condensation catalyst and unchanged starting material, can be used with excellent results. While the exact structure of these phenolic condensation products is uncertain, the Y groups linking the phenolic nuclei all fall into the preferred subgenus 1. For method of preparation, see e.g., U.S. patent No. 3,124,555, U.S. patent No. 3,242,135, and British patent No. 961,504.

When the stabilizer composition is used in conjunction with a polyvalent metal salt of an organic acid, the organic acid will ordinarily have from about six to about twenty-four carbon atoms. The polyvalent metal can be any metal of Group II of the Periodic Table, such as zinc, calcium, cadmium, barium, magnesium and strontium. The alkali metal salts and heavy metal salts such as lead salts are unsatisfactory. The acid can be any organic non-nitrogenous monocarboxylic acid having from six to twenty-four carbon atoms. The aliphatic, aromatic, alicyclic and oxygen-containing heterocyclic organic acids are operable as a class. By the term "aliphatic acid" is meant any open chain carboxylic acid, substituted, if desired, with nonreactive groups, such as halogen, sulfur and hydroxyl. By the term "alicyclic" it will be understood that there is intended any cyclic acid in which the ring is nonaromatic and composed solely of carbon atoms, and such acids may if desired have inert, nonreactive substituents such as halogen, hydroxyl, alkyl radicals, alkenyl radicals and other carbocyclic ring structures condensed therewith. The oxygen-containing heterocyclic compounds can be aromatic or nonaromatic and can include oxygen and carbon in the ring structure, such as alkyl-substituted furoic acid. The aromatic acids likewise can have nonreactive ring substituents such as halogen, alkyl and alkenyl groups, and other saturated or aromatic rings condensed therewith.

As exemplary of the acids which can be used in the form of their metal salts there can be mentioned the following: hexoic acid, 2-ethylhexoic acid, n-octoic acid, isooctoic acid, capric acid, undecylic acid, lauric acid, myristic acid, palmitic acid, margaric acid, stearic acid, oleic acid, ricinoleic acid, behenic acid,

chlorocaproic acid, hydroxy capric acid, benzoic acid, phenylacetic acid, butyl benzoic acid, ethyl benzoic acid, propyl benzoic acid, hexyl benzoic acid, salicylic acid, naphthoic acid, 1-naphthalene acetic acid, orthobenzoyl benzoic acid, naphthenic acids derived from petroleum, abietic acid, dihydriabietic acid, hexahydrobenzoic acid, and methyl furoic acid.

The water-insoluble salts are preferred, because they are not leached out when the plastic is in contact with water. Where these salts are not known, they are made by the usual types of reactions, such as by mixing the acid, or anhydride with the corresponding oxide or hydroxide of the metal in a liquid solvent, and heating, if necessary, until salt formation is complete.

A variety of organic triphosphites and acid phosphites can be employed, of which the following are exemplary.

The organic triphosphite can be any organic phosphite having three or more organic radicals attached to phosphorus through oxygen. The acid phosphite can be any organic phosphite having one or two organic radicals attached to phosphorus through oxygen. These radicals can be monovalent radicals, in the case of the triphosphites, diphosphites and monophosphites.

The organic triphosphites in which the radicals are monovalent radicals can be defined by the formula:

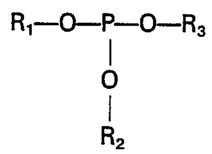

in which

$R_1$, $R_2$ and $R_3$ are selected from the group consisting of alkyl, alkenyl, aryl, alkaryl, aralkyl, and cycloalkyl groups having from one to about thirty carbon atoms.

The acid phosphites are defined by the same formula, but one or two of $R_1$, $R_2$ and $R_3$ is hydrogen or a cation of a metal or ammonium.

Also included are the organic triphosphites having a bivalent organic radical forming a heterocyclic ring with the phosphorus of the type:

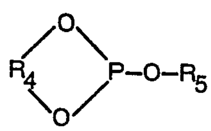

in which

$R_4$ is a bivalent organic radical selected from the group consisting of alkylene, arylene, aralkylene, alkarylene and cycloalkylene radicals having from two to about thirty carbon atoms, and $R_5$ is a monovalent organic radical as defined above in the case of $R_1$, $R_2$ and $R_3$;

$R_5$ is hydrogen or a cation, in the case of the acid phosphites.

Also useful organic triphosphites are mixed heterocyclic-open chain phosphites of the type:

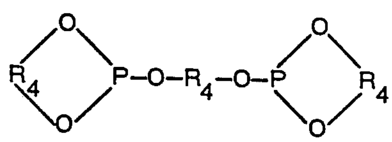

More complex triphosphites are formed from trivalent organic radicals, of the type:

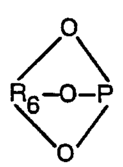

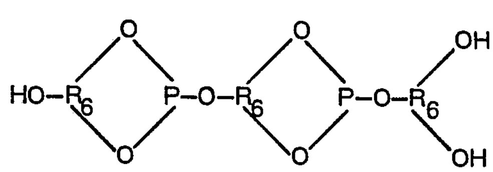

in which

$R_6$ is a trivalent organic radical of any of the types of $R_1$ to $R_5$, inclusive, as defined above.

A particularly useful class of complex triphosphites are the tetraoxadiphosphaspiro undecanes of the formula:

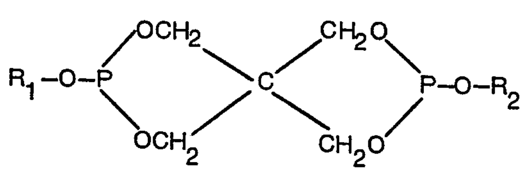

where

$R_1$ and $R_2$ are selected from the group consisting of aryl, alkyl, aryloxyethyl, alkyloxyethyl, aryloxyethyl, alkyloxyethoxyethyl and alkyloxypolyethoxyethyl having from about 1 to about 30 carbon atoms.

In the case of the acid phoshites, one or both of $R_1$ and $R_2$ is also hydrogen or a cation.

An especially preferred class of organic triphosphites and acid phosphites have a bicyclic aromatic group attached to phosphorus through oxygen, with no or one or more phenolic hydroxyl groups on either or both of the aromatic rings. These phosphites are characterized by the formula:

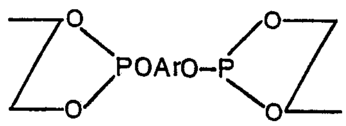

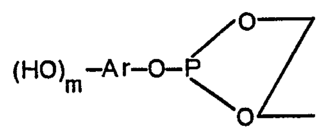

in which

Ar is a mono or bicyclic aromatic nucleus and m is an integer of from 0 to about 5. Z is one or a plurality of organic radicals as defined above for $R_1$ to $R_6$, taken singly or together in sufficient number to satisfy the valences of the two phosphite oxygen atoms.

One or both Z radicals is also hydrogen, in the case of the acid phosphites, and can include additional bicyclic aromatic groups of the type $(HO)_m$—Ar.

The cation in the case of acid phosphites can be a metal, such as an alkali metal, for instance, sodium, potassium or lithium; an alkaline earth metal, for instance, barium, calcium, or a nontoxic polyvalent metal, such as magnesium, tin and zinc.

Usually, the triphosphites and acid phosphites will not have more than about sixty carbon atoms.

Exemplary triphosphites are monophenyl di-2-ethylhexyl phosphite, diphenyl mono-2-ethylhexyl phosphite, di-isooctyl monotolyl phoshite, tri-2-ethylhexyl phosphite, phenyl dicyclohexyl phosphite, phenyl diethyl phosphite, triphenyl phosphite, tricresyl phosphite, tri(dimethylphenyl)phosphite, trioctadecyl phosphite, triisooctyl phosphite, tridodecyl phosphite, isooctyl diphenyl phosphite, diisooctyl phenyl phosphite, tri(t-octylphenyl)phosphite, tri-(t-nonylphenyl)phosphite, benzyl methyl isopropyl phosphite, butyl dicresyl phosphite, isooctyl di(octylphenyl)phoshite, di(2-ethylhexyl)(isooctylphenyl)-phosphite, tri (2-cyclohexylphenyl)phosphite), tri-α-naphthyl phosphite, tri(phenylphenyl)phosphite, tri(2-phenylethyl)phosphite, ethylene phenyl phosphite, ethylene t-butyl phosphite, ethylene isohexyl phosphite, ethylene isooctyl phosphite, ethylene cyclohexyl phosphite, 2-phenoxy-1,3,2-dioxaphosphorinane, 2-butoxy-1,3,2-dioxyphosphorinane, 2-octoxy-5,5-dimethyl-dioxaphosphorinane, and 2-cyclohexyloxy-5,5-diethyl dioxaphosphorinane.

Exemplary pentaerythritol triphosphites are 3,9-diphenoxy-2,4,8,10-tetraoxa-3,9-diphosphaspiro-(5,5)-undecane (diphenyl-pentaerythritol diphosphite), 3,9-di(decyloxy)-2,4,8,10-tetraoxa-3,9-diphosphaspiro-(5,5)-undecane, 3,9-di(isodecyloxy)-2,4,8,10-tetraoxa-3,9-diphosphaspiro-(5,5)-undecane, 3,9-di(isodecyloxy)-2,4,8,10-tetraoxa-3,9-diphosphaspiro-(5,5)-undecane, 3,9-di(octadecyloxy)-2,4,8,10-tetraoxa-3,9-diphosphaspiro-(5,5)-undecane, 3-phenoxy-9-isodecyloxy-2,4,8,10-tetraoxa-3,9-diphosphaspiro-(5,5)-undecane, 3,9-di(methoxy)-2,4,8,10-tetraoxa-3,9-diphosphaspiro-(5,5)-undecane, 3,9-di(lauryloxy)-2,4,8,10-tetraoxa-3,9-diphosphaspiro-(5,5)-undecane, 3,9-di-p-tolyloxy-2,4,8,10-tetraoxa-3,9-diphosphaspiro-(5,5)-undecane, 3,9-di(methoxyethyloxy)-2,4,8,10-tetraoxa-3,9-diphosphaspiro-(5,5)-undecane, 3-methoxyethyloxy-9-isodecyloxy-2,4,8,10-tetraoxa-3,9-diphosphaspiro-(5,5)-undecane, 3,9-di(ethoxyethyloxy)-2,4,8,10-tetraoxa-3,9-diphosphaspiro-(5,5)-undecane, 3,9-di(butoxyethyloxy)-2,4,8,10-tetraoxa-3,9-diphosphaspiro-(5,5)-undecane, 3-methoxyethyloxy-9-butoxy-ethyloxy-2,4,8,10-tetraoxa-3,9-diphosphaspiro-(5,5)-undecane, 3,9-di(methoxyethoxyethyloxy)-2,4,8,10-tetraoxa-3,9-diphosphaspiro-(5,5)-undecane, 3,9-di(butoxyethoxyethyloxy)-2,4,8,10-tetraoxa-3,9-diphosphaspiro-(5,5)-undecane, 3,9-di(methoxyethoxyethoxyethyloxy)-2,4,8,10-tetraoxa-3,9-diphosphaspiro-(5,5)-undecane, 3,9-di(methoxy(polyethoxy)-ethyloxy)-2,4,8,10-tetraoxa-3,9-diphosphaspiro-(5,5)-undecane where the (polyethoxy) ethyloxy group has an average molecuar weight of 350), 3,9-di(methoxy(polyethoxy)ethyloxy)-2,4,8,10-tetraoxa-3,9-diphosphaspiro-(5,5)-undecane (where the (polyethoxy)ethyloxy group has an average molecular weight of 550).

Exemplary of the bis aryl triphosphites are: bis(4,4′-thio-bis(2-tertiary-butyl-5-methyl-phenol))isooctyl phosphite, mono(4,4′-thio-bis(2-tertiary-butyl-5-methyl-phenol))di-phenyl phosphite, tri-(4,4′-n-butylidene-bis(2-tertiary-butyl-5-methylphenol))phosphite, (4,4′-benzylidene-bis(2-tertiary-butyl-5-methyl-phenol))-diphenyl phosphite, isooctyl 2,2′-bis(-parahydroxyphenyl)propane phosphite, decyl 4,4′-n-butylidene-bis-(2-tertiary-butyl-5-methylphenol) phosphite, tri-4,4′-thio-bis (2-tertiary-butyl-5-methylphenol)phosphite, 2-ethylhexyl-2,2′-methylene-bis(4-methyl-6,1′-methylcyclohexyl)phenol phosphite, tri(2,2′-bis-(parahydroxyphenyl)propane)phosphite, tri(4,4′-thio-bis(2-tertiary-butyl-5-methyl-phenol)phosphite, isooctyl-(2,6-bis(2′-hydroxy-3,5-dinonylbenzyl)-4-nonyl phenyl))phosphite, tetra-tridecyl-4,4′-n-butylidene-bis(2-tertiary-butyl-5-methylphenyl)diphosphite, tetra-isooctyl-4,4'-thio-bis (2-tertiary-butyl-5-methylphenyl)-diphosphite, 2,2'-methylene-bis(4-methyl-6,1'-methyl cyclohexyl phenyl)polyphosphite, isooctyl-4,4'-isopropylidene-bis-phenyl polyphosphite, 2-ethylhexyl-2,2'-methylene-bis(4-methyl-6,1'-methyl-cyclohexyl)-phenyl triphosphite, tetra-tridecyl-4,4'-oxydiphenyl diphosphite, tetra-n-dodecyl-4,4'-n-butylidene bis (2-tertiary-butyl-5-methylphenyl)diphosphite, tetra-tridecyl-4,4'-isopropylidene bisphenyl diphosphite, hexa-tridecyl butane-1,1,3-tris(2'-methyl-5'-tertiary-butylphenyl-4')triphosphite.

Exemplary acid phosphites are di(phenyl)phosphite, monophenyl phosphite, mono(diphenyl)phosphite, dicresyl phosphite, di-(o-isooctylphenyl)phosphite, di(p-ethylhexylphenyl)phosphite, di(p-t-octylphenyl)phosphite, di(dimethylphenyl)phosphite, di-n-butyl phosphite, di-2-ethylhexyl phosphite, mono-2-ethylhexylphosphite, diisooctyl phosphite, monoisooctyl phosphite, monododecyl phosphite, 2-ethylhexyl phenyl phosphite, 2-ethylhexyl-(n-octylphenyl)phosphite, monocyclohexyl phosphite, dicyclohexyl phosphite, di(2-cyclohexyl phenyl)phosphite, di-α-naphthyl phosphite, diphenyl phenyl phosphite, di(diphenyl) phosphite, di-(2-phenyl ethyl)phosphite, dibenzyl phosphite, monobenzyl phosphite, n-butyl cresyl phosphite and didodecyl phosphite, cresyl phosphite, t-octylphenyl phosphite, ethylene phosphite, butyl cresyl phosphite, isooctyl monotolyl phosphite and phenyl cyclohexyl phosphite.

Exemplary of the bis aryl acid phosphites are: bis(4,4'-thio-bis(2-tertiary-butyl-5-methylphenol))-phosphite, (4,4'-thio-bis(2-tertiary-butyl-5-methylphenyl))phenyl phosphite, bis(4,4'-n-butylidene-bis(2-tertiary-butyl-5-methylphenol))phosphite, mono(4,4'-benzylidene-bis(2-tertiary-butyl-5-methylphenol))-phosphite, mono(2,2'-bis-(parahydroxyphenyl)propane)phosphite, mono(4,4'-butylidene-bis(2-tertiary-butyl-5-methylphenol)phosphite, bis(4,4'-thio-bis(2-tertiary-butyl-5-methylphenol))phosphite, mono-2-ethylhexyl-mono-2,2'-methylene-bis(4-methyl-6,1'-methylcyclohexyl)phenol phosphite, bis(2,2'-bis(parahydroxyphenyl)propane)phosphite, monoisooctylmono(4,4'-thio-bis(2-tertiary-butyl-5-methylphenol))-phosphite, isooctyl-(2,6-bis(2'-hydroxy-3,5-dinonylbenzyl)-4-nonylphenyl))phosphite, tri-tridecyl-4,4'-n-butylidene-bis(2-tertiary-butyl-5-methylphenyl)diphosphite, triisooctyl-4,4'-thio-bis(2-tertiary-butyl-5-methylphenyl)diphosphite, bis(2,2'-methylene-bis(4-methyl-6,1'-methyl cyclohexyl phenyl))phosphite, isooctyl-4,4'-isopropylidene-bis-phenyl phosphite, monophenyl mono(2,2'-methylene-bis(4-methyl-6,1'-methyl-cyclohexyl))triphosphite, di-tridecyl-4,4'-oxydiphenyl diphosphite, di-n-dodecyl-4,4'-n-butylidene-bis(2-tertiary-butyl-5-methylphenyl)diphosphite, di-tridecyl-4,4'-isopropylidene bisphenyl diphosphite, tetra-tridecyl butane-1,1,3-tris(2'-methyl-5-tertiary-butylphenyl-4)-triphosphite. methylphenyl)diphosphite, di-tridecyl-4,4'-isopropylidene bisphenyl diphosphite, tetra-tridecyl butane-1,1,3-tris(2'-methyl-5-tertiary-butylphenyl-4)-triphosphite.

The thiodipropionic acid ester has the following formula:

$$R_1OOCCH_2CH_2—S—CH_2CH_2COOY$$

in which $R_1$ is an organic radical selected from the group consisting of hydrocarbon radicals such as alkyl, alkenyl, aryl, cycloalkyl and mixed alkyl aryl and mixed alkyl cycloalkyl radicals; hydroxyalkyl and hydroxyalkyloxyalkyklene radicals; and esters thereof with aliphatic carboxylic acids; and Y is selected from the group consisting of (a) hydrogen, (b) a second R radical $R_2$, which can be the same or different from the $R_1$ radical, (c) a polymeric chain of n thiodipropionic acid ester units:

$$—XO[OCCH_2CH_2SCH_2CH_2COOXO]_nOCCH_2CH_2—S—CH_2CH_2COOZ$$

where Z is hydrogen, $R_2$ or M, n is the number of thiodipropionic acid ester units in the chain, and X is a bivalent hydrocarbon group of the type of $R_1$, that is, alkylene, alkenylene, cycloalkylene, mixed alkylenearylene and mixed alkylenecycloalkylene radicals; hydroxyalkylene and hydroxyalkyloxyalkylene radicals; and esters thereof with aliphatic carboxylic acids; the value of n can range upwards from 0, but there is no upper limit on n except as is governed by the ratio of carbon atoms to sulfur atoms as stated below; and (d) a polyvalent metal M of Group II of the periodic table such as zinc, calcium, cadmium, barium and strontium.

The molecular weights of the R and Y radicals are taken such that with the remainder of the molecule the thiodipropionic ester has a total of from about ten to about sixty carbon atoms per sulfur atom.

Accordingly, the various thiodipropionic acid ester species coming within the above-designated categories within the general formula can be defined as follows:

(a) $R_1OOCCH_2CH_2SCH_2CH_2COOH$
(b) $R_1OOCCH_2CH_2SCH_2CH_2COOR_2$
(c) $R_1O[OCCH_2CH_2SCH_2CH_2COOX—O]_nOCCH_2CH_2SCH_2CH_2COOZ$
(d) $R_1OOCCH_2CH_2SCH_2CH_2COOM$

In the above formulae $R_1$ and $R_2$, M, X and Z are the same as before and the value of $n_1$ can range upwards from 1, but there is no upper limit on $n_1$ except as is imposed by the ratio of carbon atoms, as stated below. In the polymer (c), as in the other forms of thiodipropionic acid esters, the total number of carbon atoms per sulfur atom is within the range from about ten to about sixty.

The R radical of these esters is important in furnishing compatibility with the polymer. The Y radical is desirably a different radical, $R_2$ or M or a polymer, where R is rather low in molecular weight, so as to compensate for this in obtaining the optimum compatibility and nonvolatility. Where Y is a metal, the thiodipropionic acid ester furnishes the beneficial properties of the polyvalent metal salt which is described above.

The aryl, alkyl, alkenyl, and cycloalkyl groups may, if desired, contain inert, nonreactive substituents such as halogen and other carbocyclic and heterocyclic ring structures condensed therewith.

Typical R radicals are, for example, methyl, ethyl, propyl, isopropyl, butyl, isobutyl, t-butyl, amyl, isoamyl, n-octyl, isooctyl, 2-ethyl hexyl, t-octyl, decyl, dodecyl, octadecyl, allyl, hexenyl, linoleyl, ricinoleyl, oleyl, phenyl, xylyl, tolyl, ethylphenyl, naphthyl, cyclohexyl, benzyl, cyclopentyl, methylcyclohexyl, ethylcyclohexyl, and naphthenyl, hydroxyethyl, hydroxypropyl, glyceryl, sorbityl, pentaerythrityl, and polyoxyalkylene radicals such as those derived from diethylene glycol, triethylene glycol, polyoxypropylene glycol, polyoxyethylene glycol, and polyoxypropyleneoxyethylene glycol, and esters thereof with any of the organic acids named below in the discussion of the polyvalent metal salts, including in addition those organic acids having from two to five carbon atoms, such as acetic, propionic, butyric and valeric acids.

Typical X radicals are alkylene radicals such as ethylene, tetramethylene, hexamethylene, decamethylene, alkyl-substituted alkylene radicals such as 1,2-propylene,

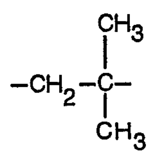

and

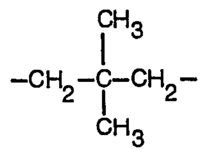

arylene radicals such as phenylene

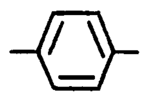

methylenephenylene

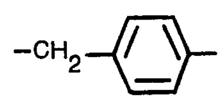

dimethylene phenylene

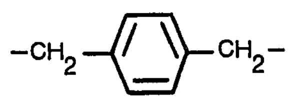

and alicyclylene such as cyclohexylene

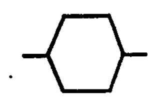

and cyclopentylene

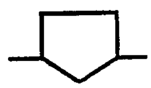

As exemplary of the thiodipropionic acid esters which can be used, there can be mentioned the following: monolauryl thiodipropionic acid, dilauryl thiodipropionate, butyl stearyl thiodipropionate, 2-ethylhexyl lauryl thiodipropionate, di-2-ethylhexyl-thiodipropionate, diisodecyl thiodipropionate, isodecyl phenyl thiodipropionate, benzyl lauryl thiodipropionate, benzyl phenyl thiodipropionate, the diester of mixed coconut fatty alcohols and thiodipropionic acid, the diester of mixed tallow fatty alcohols and thiodipropionic acid, the acid ester of mixed cottonseed oil fatty alcohols and thiodipropionic acid, the acid ester of mixed soyabean oil fatty alcohols and thiodipropionic acid, cyclohexyl nonyl thiodipropionate, monooleyl thiodipropionic acid, hydroxyethyl lauryl thiodipropionate, monoglyceryl thiodipropionic acid, glyceryl monostearate monothiodipropionate, sorbityl isodecyl thiodipropionate, the polyester of diethylene glycol and thiodipropionic acid, the polyester of triethylene glycol and thiodiopropionic acid, the polyester of hexamethylene glycol and thiodipropionic acid, the polyester of pentaerythritol and thiodipropionic acid, the polyester of octamethylene glycol and thiodipropionic acid, the polyester of p-dibenzyl alcohol and thiodipropionic acid, ethylbenzyl lauryl thiodipropionate, strontium stearyl thiodipropionate, magnesium oleyl thiodipropionate, calcium dodecylbenzyl thiodipropionate, and mono(dodecylbenzyl)thiodipropionic acid.

These esters are for the most part known compounds, but where they are not available, they are readily prepared by esterification of thiodipropionic acid and the corresponding alcohol.

Also useful are:

(1) Thioalkanoic acid amides of Tokuno et al Japanese patent No. 16,286/68 having the formula:

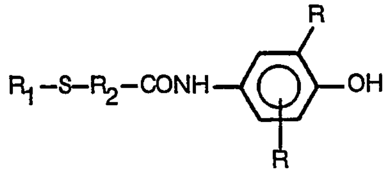

R is alkyl of one to eight carbon atoms, $R_1$ is alkyl of six to twenty-four carbon atoms, and $R_2$ is alkylene of one to six carbon atoms.

(2) Thioalkanoic acid amides of 1,3,5-triazines of Ozeki et al Japanese patent No. 20,366/68 having the formula:

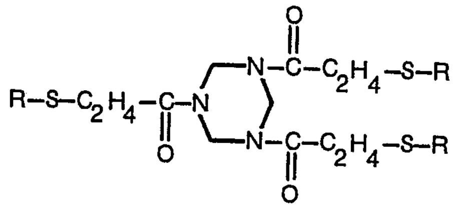

R is alkyl of eight to eighteen carbon atoms.

(3) Bis-thioalkanoic acid amides of Yamamotor et al Japanese patent No. 23,765/68 having the formula:

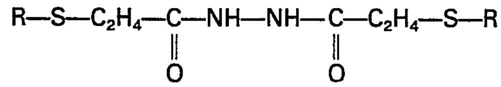

R is alkyl of more than six carbon atoms, aryl or aralkyl.

(4) Bis-thioalkylanoic acid amides of Ozeki et al Japanese patent No. 26,184/69 having the formula:

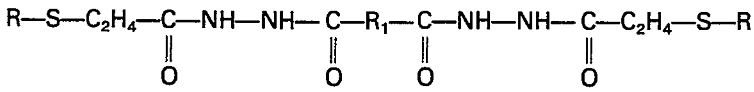

R is alkyl of twelve to eighteen carbon atoms, and $R_1$ is alkylene of one to ten carbon atoms, cycloalkylene, or arylene.

(5) Bis-alkylene thioalkanoic acid amides of Ozeki Japanese patent No. 31,464/69 having the formula:

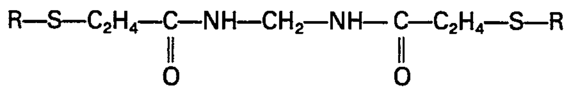

R is alkyl of more than six carbon atoms, aryl, or aralkyl.

(6) Thioalkanoic acid amide derivatives of Minagawa et al, published Japanese application No. 106,484/74 having the formula:

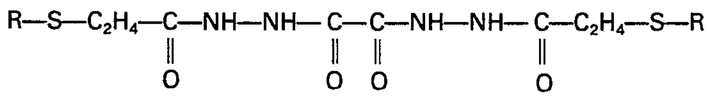

R is hydrocarbyl of one to twenty carbon atoms.

(7) Alkylene bis-thioalkanoic acid amides of U.S. patent No. 4,279,805 to Ohzeki et al, patented July 21, 1981, having the general formula:

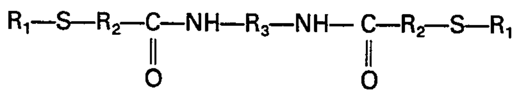

wherein:

$R_1$ is alkyl having from one to about fifty carbon atoms;
$R_2$ is alkylene having from one to about three carbon atoms; and
$R_3$ is alkylene having from about two to about twelve carbon atoms.

β-Alkylthiopropionic acid esters having the general formula:

$$R—S—C_2H_4COOR—(R')_n$$

wherein:

R is alkyl of four to twenty carbon atoms;
n is a number from 1 to 6; and

R' is the residue of an alcohol having from one to six hydroxyl groups.

Pentaerythritol tetra dodecyl thio propionate is an example of this group.

Other conventional light stabilizers can be employed, such as hydroxybenzophenones such as 2-hydroxy-4-methoxybenzophenone, 2-hydroxy-4-n-octoxy benzophenone, 2,4-dihydroxybenzophenone, benzotriazoles, such as 2(2-hydroxy-5-methylphenyl)benzotriazoles, 2(2-hydroxy-3-t-butyl-5-methylphenyl)-5-chlorobenzotriazole, 2(2-hydroxy-3,5-di-t-butylphenyl) 5-chlorobenzotriazole, 2(2-hydroxy-3,5-di-t-amylphenyl)benzotriazole, benzoates such as phenylsalicylate, 2,4-di-t-butylphenyl-3,5-di-t-butyl-4-hydroxy phenylbenzoate, nickel compounds such as nickel 2,2'-thiobis(4-t-octyl phenolate), nickel mono ethyl (3,5-di-t-butyl 4-hydroxybenzyl)phosphonate, substituted acrylonitriles such as methyl-α-cyano β-methyl-β-(p-methoxy phenyl)acrylate and oxalic anilides such as N-2 ethyl phenyl-N'-2 ethoxy 5-t butyl phenyl oxalic diamide, N-2-ethyl phenyl-N'-2 ethoxy phenyl oxalic diamide.

A sufficient amount of the stabilizer or combination is used to improve the resistance of the polyvinyl chloride to deterioration in physical properties when exposed to heat and light, including, for example, discoloration and embrittlement. Very small amounts are usually adequate. Amounts within the range from about 0.001 to about 10% total stabilizers including the oxirane of the invention by weight of the polymer are satisfactory. Preferably, from 0.01 to 5% is employed for optimum stabilization.

The stabilizer systems of the invention are readily formulated as a blend of:

(a) α-hydroxy-β-2,2,6,6-tetramethyl piperidyl ethylene oxirane in an amount of from about 10 to about 35 parts by weight; and optionally:

(b) a phenolic antioxidant in an amount from about 10 to about 35 parts by weight; and/or

(c) other heat or light stabilizers in an amount of from about 10 to about 35 parts by weight.

In addition, other conventional additives for polyvinyl chloride resins, such as antistatic agents, flameproofing agents, pigments and fillers, can be employed.

The stabilizer or combination is incorporated in the polymer in suitable mixing equipment, such as a mill or a Banburn mixer. If the polymer has a melt viscosity which is too high for the desired use, the polymer can be worked until its melt viscosity has been reduced to the desired range before addition of the stabilizer. Mixing is continued until the mixture is substantially uniform. The resulting composition is then removed from the mixing equipment and brought to the size and shape desired for marketing or use.

The stabilized polymer can be worked into the desired shape, such as by milling, calendering, extruding or injection molding of fiber-forming. In such operations, it will be found to have a considerably improved resistance to reduction in melt viscosity during the heating, as well as a better resistance to discoloration and embrittlement on ageing and heating.

The following Examples represent preferred embodiments of polyvinyl chloride resin compositions in accordance with the invention.

## Examples 1 to 7

Rigid polyvinyl chloride resin compositions were prepared having the following formulation:

| Ingredient | Parts by Weight |
|---|---|
| Polyvinyl chloride homopolymer | 100 |
| Stearyl alcohol | 0.5 |
| Polyethylenewas(Hiwax405MP:MitsuiPetrochem.) | 0.3 |
| 1,4-Butanediol-bis(β-aminocrotonate) | 1.0 |
| Stabilizer as shown in Table I | 0.3 to 3.3 as shown in the Table |

The compositions were thoroughly blended on a two-roll mill, and sheeted off to form sheets 1 mm thick. Pieces were cut off from the sheets, and heated at 190°C in a Geer oven to determine heat stability, as the time required for the pieces to turn black. The initial color of the sheets and the color of the test pieces after heating for 60 minutes at 175°C were also noted, and evaluated according to the following scale:

| Color Scale | Color |
|---|---|
| 1 | Colorless |
| 2 | Pale yellow tint |
| 3 | Pale yellow |
| 4 | Pale to light yellow |
| 5 | Light yellow |
| 6 | Light to medium yellow |
| 7 | Medium yellow |
| 8 | Dark yellow |
| 9 | Yellow and black |
| 10 | Black |

The results are shown in Table I.

TABLE I

| Example No. | Stabilizer (phr) | Heat Stability at 190°C (minutes) | Initial Color | Color after Heating |
|---|---|---|---|---|
| Control 1 | Bisphenol A diglycidylether (3.0) | 60 | 8 | 6 |
| Control 2 | { Bisphenol A diglycidylether (3.0)<br>Tetra(2,2,6,6-tetramethyl-4-piperidyl)-1,2,<br>3,4-butanetetracarboxylate (0.3) } | 75 | 8 | 7 |
| Control 3 | Tris(2,3-epoxypropyl)isocyanurate (1.0) | 75 | 6 | 4 |
| Control 4 | { Tris(2,3-epoxypropyl)isocyanurate (1.0)<br>Bis(2,2,6,6-tetramethyl-4-piperidyl)<br>sebacate (0.5) } | 90 | 7 | 6 |
| Example 1 | Example II<br>Reaction product of tris(2,3-epoxypropyl) isocyanurate (50 g) and tetra(2,2,6,6-tetramethyl 4-piperidyl-1,2,3,4-butanetetracarboxylate (25 g) (1.5) | 105 | 2 | 1 |
| Example 2 | Example IV<br>Reaction product of Bisphenol A diglycidyl-ether (20 g) with tetra(2,2,6,6-tetramethyl-4-piperidyl) 1,2,3,4-butanetetracarboxylate (2g) (3.3) | 90 | 4 | 2 |
| Example 3 | Example VIII<br>Reaction product of 3,4-epoxycyclohexylmethyl 3,4-epoxycyclohexanecarboxylate (30 g) with tetra(2,2,6,6-tetramethyl-4-piperidyl)-1,2,3,4 butanetetracarboxylate (3 g) (3,3) | 90 | 4 | 2 |

TABLE I (continued)

| Example No. | Stabilizer (phr) | Heat Stability at 190°C (minutes) | Initial Color | Color after Heating |
|---|---|---|---|---|
| Example 4 | Example X<br>Reaction product of 3,4-epoxycyclohexylmethyl-3,4-epoxycyclohexanecarboxylate (10 g) with tetra(2,2,6,6-tetramethyl-4-piperidyl) 1,2,3,4-butanetetracarboxylate (2 g) (1.8) | 90 | 4 | 2 |
| Example 5 | Example XIII<br>Reaction product of Bisphenol A diglycidylether (30g) with bis(2,2,6,6-tetramethyl-4-piperidyl)-di(tridecyl)-1,2,3,4-butanetetracarboxylate (3 g) (3.3) | 75 | 4 | 2 |
| Example 6 | Example XIX<br>Reaction product of Bisphenol A diglycidylether (30g) with 3,9 bis(1,1-dimethyl 2(tris(2,2,6,6-tetramethyl 4 piperidyloxycarbonyl)butylcarbonyloxy)ethyl) 2,4,8,10-tetraoxaspiro[5.5]undecane (3 g) (3.3) | 90 | 4 | 2 |
| Example 7 | Example XXIV<br>Reaction product of tris(2,3-epoxypropyl)isocyanurate (20 g) with bis(2,2,6,6-tetramethyl-4 piperidyl) sebacate (10 g) (1.5) | 105 | 3 | 2 |

It is apparent from the results that the oxiranes in accordance with the invention are superior to the controls, the unreacted oxirane or piperidyl compound, taken singly or in admixture, in improving the resistance of the polyvinyl chloride resin to deterioration when exposed to heat.

Examples 8 to 13

Rigid polyvinyl chloride resin compositions were prepared according to the following formulation:

| Ingredient | Parts by Weight |
|---|---|
| Polyvinyl chloride resin homopolymer | 100 |
| Stearyl alcohol | 0.3 |
| Polyethylene wax | 0.2 |
| Dioctyltinbis(2-ethylhexylthioglycolate) | 2.0 |
| Stabilizer as shown in Table II | 0.4 to 2.1 as shown in the Table |

The compositions were thoroughly blended on a two-roll mill and sheeted off to form sheets 1 mm thick. Test pieces were cut off from the sheets and heated at 190°C in a Geer oven until black, to determine heat stability.

Weatherability was also evaluated by exposing test pieces from the sheets for six months in the open air. The initial color of the pieces and the color of the test pieces after weathering were noted. Weatherability was rated according to the same color scale indicated above.

The results are shown in Table II.

TABLE II

| Example No. | Stabilizer (phr) | Heat Stability at 190°C (minutes) | Initial Color | Weatherability |
|---|---|---|---|---|
| Control 1 | None | 60 | 1 | 8 |
| Control 2 | Bisphenol A diglycidylether (1.5) | 90 | 2 | 7 |
| Control 3 | Bisphenol A diglycidylether (1.5)<br>Bis(2,2,6,6-tetramethyl-4-piperidyl) sebacate (0.1) | 105 | 3 | 6 |
| Control 4 | Tris(2,3-epoxypropyl)isocyanurate (1.0) | 75 | 1 | 7 |
| Control 5 | Tris(2,3-epoxypropyl)isocyanurate (1.0)<br>Tetra(2,2,6,6-tetramethyl-4 piperidyl 1,2,3,4-butanetetracarboxylate (0.4) | 105 | 3 | 5 |
| Example 8 | Example I<br>Reaction product of Bisphenol A diglycidylether (50 g) with tetra(2,2,6,6-tetramethyl 4-piperidyl)-1,2,3,4-tetracarboxylate (10 g) (1.8) | 120 | 1 | 3 |
| Example 9 | Example VI<br>Reaction product of tris(2,3-epoxypropyl) isocyanurate (50 g) with tetra(2,2,6,6-tetramethyl-4 piperidyl)-1,2,3,4 butane tetracarboxylate (20 g) (1.4) | >120 | 1 | 2 |

TABLE II (continued)

| Example No. | Stabilizer (phr) | Heat Stability at 190°C (minutes) | Initial Color | Weatherbility |
|---|---|---|---|---|
| Example 10 | Example XI<br>Reaction product of epoxidized-1,2-poly-butadiene (Adeka Argus Chemical Co., Ltd. BF 1000; M.W.=1000, oxirane oxygen=7.0%) (20 g) with tetra(2,2,6,6-tetramethyl 4-piperidyl)-1,2,3,4 butanetetracarboxylate (1 g) (2.1) | 120 | 2 | 3 |
| Example 11 | Example XVII<br>Reaction product of 3,4-epoxycyclohexylmethyl-3,4 epoxycyclohexanecarboxylate (10 g) with bis(2,2,6,6-tetramethyl-4-piperidyl)-di(tridecyl) 1,2,3,4 butanetetracarboxylate (2 g) (1.2) | 120 | 2 | 3 |
| Example 12 | Example XXIII<br>Reaction product of Bisphenol A diglycidylether (30 g) with bis(2,2,6,6-tetramethyl-4 piperidyl) sebacate (2 g) (1,6) | 120 | 1 | 3 |
| Example 13 | Example XXV<br>Reaction product of Bisphenol A diglycidylether (30 g) with N-(2,2,6,6-tetramethyl 4-piperidyl) dodecylsuccinimide (2 g) (1.6) | 120 | 2 | 4 |

It is apparent from the results that the oxiranes in accordance with the invention are superior to the controls, the unreacted oxirane or piperidyl compound, taken singly or in admixture, in improving the resistance of the polyvinyl chloride resin to deterioration when exposed to heat and ultraviolet light.

Examples 14 to 25

Polyvinyl chloride resin compositions plasticized with dioctyl phthalate and tricresol phosphate were prepared according to the following formulation:

| Ingredient | Parts by Weight |
|---|---|
| Polyvinyl chloride homopolymer | 100 |
| Dioctyl phthalate | 50 |
| Tricresyl phosphate | 5 |
| Sorbitan monostearate | 2 |
| Methylene-bis-(stearic amide) | 0.3 |
| Ca stearate | 0.3 |
| Zn stearate | 0.8 |
| Stabilizer as shown in Table III | 0.1 to 2.8 as shown in the Table |

The compositions were thoroughly blended on a two roll mill and then compression-molded to form sheets 1 mm thick. Pieces were cut off from the sheets and heated at 190°C in a Geer oven until black to determine heat stability.

Test pieces were also exposed to the open air for fourteen months to determine weatherability. The initial color of the pieces and the color of the test pieces after weathering was also rated according to the color scale above, and the results are shown in Table III.

TABLE III

| Example No. | Stabilizer (phr) | Heat Stability at 190°C (minutes) | Initial Color | Weatherability |
|---|---|---|---|---|
| Control 1 | Bisphenol A diglycidylether (2.0) | 60 | 4 | 7 |
| Control 2 | { Bisphenol A diglycidylether (2.0) Tetra(2,2,6,6-tetramethyl-4-piperidyl)-1,2,3,4-butanetetracarboxylate (0.1) } | 75 | 5 | 6 |
| Example 14 | Example I<br>Reaction product of Bisphenol A diglycidyl ether [illegible] g) with tetra(2,2,6,6-tetramethyl-4 pyridyl)-1,2,3,4 tetracarboxylate (10 g) (2.4) | 105 | 4 | 2 |
| Example 15 | Example III<br>Reaction product of Bisphenol A diglycidyl ether (20 g) with tetra(2,2,6,6 tetramethyl 4-piperidyl) 1,2,3,4-butanetetracarboxylate (1 g) (2.1) | 90 | 3 | 4 |
| Example 16 | Example IV<br>Reaction product of Bisphenol A diglycidyl-ether (20 g) with tetra(2,2,6,6-tetramethyl-4-piperidyl)-1,2,3,4-butanetetracarboxylate(2g)(2.2) | 90 | 3 | 3 |
| Example 17 | Example V<br>Reaction product of Bisphenol A diglycidyl-ether (20 g) with tetra(2,2,6,6 tetramethyl 4-piperidyl)-1,2,3,4-butanetetracarboxylate(3g)(2.3) | 105 | 4 | 3 |
| Example 18 | Example VIII<br>Reaction product of 3,4-epoxycyclohexylmethyl 3,4-epoxycyclohexanecarboxylate (30 g) with tetra(2,2,6,6-tetramethyl-4-piperidyl)-1,2,3,4-butanetetracarboxylate (3 g) (2.2) | 90 | 4 | 4 |
| Example 19 | Example IX<br>Reaction product of epoxidized-1,2-polybutadiene (Adeka Argus Chemical Co., Ltd. BF-1000; M.W. = 1000, oxirane oxygen = 7.0%) (10 g) with tetra(2,2,6,6-tetramethyl-4-piperidyl)-1,2,3,4 butanetetracarboxylate (4 g) (2.8) | 105 | 5 | 3 |

TABLE III (continued)

| Example No. | Stabilizer (phr) | Heat Stability at 190°C (minutes) | Initial Color | Weatherability |
|---|---|---|---|---|
| Example 20 | Example XII<br>Reaction product of epoxidized 1,2-polybutadiene (Adeka Argus Chemical Co., Ltd. BF-1000; M.W. = 1000, oxirane oxygen = 7.0%) (20 g) with tetra(2,2,6,6-tetramethyl 4-piperidyl)-1,2,3,4-butanetetracarboxylate (2 g) (2.2) | 90 | 4 | 4 |
| Example 21 | Example XIV<br>Reaction product of Bisphenol A diglycidylether (30g) with bis(2,2,6,6-tetramethyl-4-piperidyl)-di(tridecyl) 1,2,3,4 butanetetracarboxylate (4 g) (2.27) | 105 | 4 | 3 |
| Example 22 | Example XVI<br>Reaction product of tris(2,3-epoxypropyl)isocyanurate (50 g) with bis(2,2,6,6-tetramethyl-4 piperidyl) di (tridecyl)-1,2,3,4-butanetetracarboxylate (5 g) (2.1) | 105 | 3 | 4 |
| Example 23 | Example XX<br>Reaction product of Bisphenol A diglycidylether (10 g) with 3,9 bis(1,1-dimethyl-2 (tris(2,2,6,6-tetramethyl-4 piperidyloxycarbonyl)butylcarbonyloxy)ethyl)-2,4,8,10-tetraoxaspiro [5.5] undecane (4 g) (2.27) | 105 | 3 | 3 |
| Example 24 | Example XXII<br>Reaction product of Bisphenol A diglycidylether (30 g) with bis(2,2,6,6-tetramethyl 4-piperidyl) sebacate (1.5 g) (2.1) | 90 | 3 | 4 |
| Example 25 | Example XXVI<br>Reaction product of Bisphenol A diglycidylether (30 g) with N-(2,2,6,6-tetramethyl 4-piperidyl) dodecylsuccinimide (3 g) (2.2) | 90 | 4 | 4 |

It is apparent from the results that the oxiranes in accordance with the invention are superior to the controls, the unreacted oxirane or piperidyl compound, taken singly or in admixture, in improving the resistance of the polyvinyl chloride resin to deterioration when exposed to heat and ultraviolet light.

Examples 26 to 32

Plasticized polyvinyl chloride resin compositions were prepared according to the following formulation:

| Ingredient | Parts by Weight |
|---|---|
| Polyvinyl chloride homopolymer | 100 |
| Dioctyl phthalate | 50 |
| Ditridecyl-phenylphosphite | 0.5 |
| Ba stearate | 1.0 |
| Zn stearate | 0.5 |
| Stabilizer as shown in Table IV | 0.1 to 2.1 as shown in the Table |

The compositions were thoroughly blended on a two-roll mill and sheets 1 mm thick were prepared by compression molding. Pieces were cut off the sheets and heated at 190°C in a Geer oven until black to determine heat stability. The initial color of the sheet pieces and the color of the test pieces after heating for 60 minutes at 175°C were also noted. The results are shown in Table IV.

TABLE IV

| Example No. | Stabilizer (phr) | Heat Stability at 190°C (minutes) | Initial Color | Color after Heating |
|---|---|---|---|---|
| Control 1 | Bisphenol A diglycidylether (2.0) | 75 | 5 | 5 |
| Control 2 | Bisphenol A diglycidylether (2.0)<br>Tetra(2,2,6,6-tetramethyl-4-piperidyl) 1,2,3,4-butanetetracarboxylate (0.1) | 90 | 6 | 7 |
| Example 26 | Example III<br>Reaction product of Bisphenol A diglycidylether (20 g) with tetra(2,2,6,6-tetramethyl-4-piperidyl)-1,2,3,4-butanetetracarboxylate (1 g) (2.1) | 105 | 3 | 3 |
| Example 27 | Mixture of Example VI with Control 1<br>Reaction product of tris(2,3-epoxypropyl) isocyanurate (50 g) with tetra(2,2,6,6-tetramethyl-4-piperidyl)-1,2,3,4 butane-tetracarboxylate (20 g) (0.35)<br>Bisphenol A diglycidylether (1.75) | 105 | 3 | 2 |
| Example 28 | Mixture of Example XIII and Control 1<br>Reaction product of Bisphenol A diglycidyl-ether (30 g) with bis(2,2,6,6-tetramethyl-4 piperidyl)-di(tridecyl)-1,2,3,4-butane-tetracarboxylate (3 g) (1.1)<br>Bisphenol A diglycidylether (1.0) | 90 | 2 | 2 |

TABLE IV (continued)

| Example No. | Stabilizer (phr) | Heat Stability at 190°C (minutes) | Initial Color | Color after Heating |
|---|---|---|---|---|
| Example 29 | Mixture of Example XVIII and Control 1<br>Reaction product of epoxidized 1,2-polybutadiene (Adeka Argus Chemical Co., Ltd. BF-1000; M.W. = 1000, oxirane oxygen = 7.0%) (10 g) with bis(2,2,6,6-tetramethyl-4-piperidyl) di(tridecyl)-1,2,3,4-butanetetracarboxylate (4 g) (0.35)<br>Bisphenol A diglycidylether (1.75) | 90 | 3 | 3 |
| Example 30 | Mixture of Example XXI and Control 1<br>Reaction product of tris(2,3-epoxypropyl) isocyanurate (20 g) with 3,9 bis(1,1-dimethyl-2-(tris(2,2,6,6-tetramethyl 4-piperidyloxycarbonyl)butylcarbonyloxy)ethyl) 2,4,8,10-tetraoxaspiro [5.5] undecane (20 g) (0.2)<br>Bisphenol A diglycidylether (1.9) | 105 | 3 | 3 |
| Example 31 | Example XXII<br>Reaction product of Bisphenol A diglycidylether (30 g) with bis(2,2,6,6-tetramethyl-4 piperidyl) sebacate (1.5 g) (2.1) | 90 | 2 | 3 |
| Example 32 | Mixture of Example XXVI and Control 1<br>Reaction product of Bisphenol A diglycidylether (30 g) with N-(2,2,6,6-tetramethyl 4-piperidyl) dodecylsuccinimide (3 g) (1.1)<br>Bisphenol A diglycidylether (1.0) | 90 | 3 | 4 |

It is apparent from the results that the oxiranes in accordance with the invention are superior to the controls, the unreacted oxirane or piperidyl compound, taken singly or in admixture, in improving the resistance of the polyvinyl chloride resin to deterioration when exposed to heat.

## Claims

1. α-Hydroxyethyl-β-2,2,6,6-tetra methyl piperidyl ethylene oxiranes, being the reaction products of a compound having the formula Ia

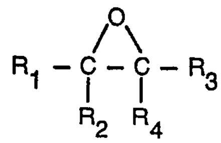

in which at least one of the groups $R_1$, $R_2$, $R_3$ and $R_4$ includes an oxirane group, the total of such oxirane groups in $R_1$, $R_2$, $R_3$ and $R_4$ being within the range of one to twenty and $R_1$, $R_2$, $R_3$ and $R_4$ being selected from the group consisting of hydrogen, aliphatic, aromatic, mixed aromatic aliphatic cycloaliphatic and mixed cycloaliphatic aliphatic hydrocarbon groups having from one to thirty carbon atoms and heterocyclic groups having from one to three ring nitrogen and/or oxygen atoms and five to three ring carbon atoms and a 2,2,6,6-tetramethylpiperidyl compound containing in the molecule the residue of formula Ib

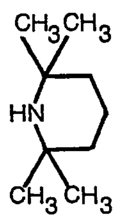

to produce a compound in which said 2,2,6,6-tetramethyl piperidyl residue becomes linked to one carbon of the oxirane ring of the compound of formula Ia and the epoxy oxygen becomes a hydroxyl group attached to the other carbon atoms of the oxirane ring.

2. α-Hydroxy-β-2,2,6,6-tetramethyl piperidylethylene oxiranes according to claim 1 in which $R_1$, $R_2$, $R_3$ and $R_4$ are aliphatic hydrocarbon groups, of which at least one includes an oxirane group.

3. α-Hydroxy-β-2,2,6,6-tetramethyl piperidyl ethylene oxiranes according to claim 1 in which $R_1$, $R_2$, $R_3$ and $R_4$ are selected from the group consisting of saturated and unsaturated aliphatic and mixed aliphatic aromatic hydrocarbon groups and such groups including at least one oxirane group.

4. A stabilizer composition capable of improving the resistance to deterioration of polyvinyl chloride resins when exposed to heat and ultraviolet light, comprising an α-hydroxy β-2,2,6,6-tetramethyl piperidyl ethylene oxirane according to one or more of the claims 1—3 and a polyvinyl chloride resin heat stabilizer.

5. A polyvinyl chloride resin stabilizer composition according to claim 4 in which the polyvinyl chloride resin heat stabilizer is a phenolic antioxidant.

6. A polyvinyl chloride resin stabilizer composition according to claim 4 in which the polyvinyl chloride resin heat stabilizer is an organic phosphite.

7. A polyvinyl chloride resin stabilizer composition according to claim 4 in which the polyvinyl chloride resin heat stabilizer is an organotin compound.

8. A polyvinyl chloride resin stabilizer composition according to claim 4 in which the polyvinyl chloride resin heat stabilizer is a thioether.

9. A polyvinyl chloride resin stabilizer composition having improved resistance to deterioration when exposed to heat and light comprising a polyvinyl chloride resin and a stabilizer composition according to claim 4.

## Patentansprüche

1. α-Hydroxyethyl-β-2,2,6,6-tetramethylpiperidylethylenoxirane, die die Reaktionsprodukte einer Verbindung der Formel Ia

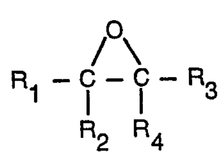

in welcher mindestens eine der Gruppen $R_1$, $R_2$, $R_3$ und $R_4$ eine Oxirangruppe umfaßt, wobei die Gesamtzahl solcher Oxirangruppen in $R_1$, $R_2$, $R_3$ und $R_4$ im Bereich von 1 bis 20 liegt und $R_1$, $R_2$, $R_3$ und $R_4$ aus der Gruppe ausgewählt sind, die aus Wasserstoff, aliphatischen, aromatischen, gemischt aromatischaliphatischen, cycloaliphatischen und gemischt cycloaliphatisch-aliphatischen Kohlenwasserstoffgruppen

mit 1 bis 30 Kohlenstoffatomen und heterocyclischen Gruppen mit 1 bis 3 Ringstickstoff- und/oder -sauerstoffatomen und 5 bis 3 Ringkohlenstoffatomen besteht, und einer 2,2,6,6-Tetramethylpiperidylverbindung sind, die im Molekül den Rest der Formel Ib

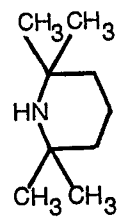

enthält, wobei in den Reaktionsprodukten der 2,2,6,6-Tetramethylpiperidylrest an das eine Kohlenstoffatom des Oxiranringes der Verbindung von Formel Ia gebunden ist und der Epoxysauerstoff zu einer Hydroxylgruppe geworden ist, die an das andere Kohlenstoffatom des Oxiranringes gebunden ist.

2. α-Hydroxy-β-2,2,6,6-tetramethylpiperidylethylen-oxirane gemäß Anspruch 1, in welchen $R_1$, $R_2$, $R_3$ und $R_4$ aliphatische Kohlenwasserstoffgruppen sind, von denen mindestens eine eine Oxirangruppe umfaßt.

3. α-Hydroxy-β-2,2,6,6-tetramethylpiperidylethylen-oxirane gemäß Anspruch 1, in welchen $R_1$, $R_2$, $R_3$ und $R_4$ aus der Gruppe ausgewählt sind, die aus gesättigten und ungesättigten aliphatischen und gemischt aliphatisch-aromatischen Kohlenwasserstoffgruppen und solchen Gruppen besteht, die mindestens eine Oxirangruppe umfassen.

4. Stabilisatorzusammensetzung zum Verbessern der Beständigkeit von Polyvinylchloridharzen gegen Zersetzung bei Einwirkung von Wärme und UV-Licht, die ein α-Hydroxy-β-2,2,6,6-tetramethylpiperidylethylen-oxiran gemäß einem oder mehreren der Ansprüche 1 bis 3 und einen Polyvinylchloridharz-Wärmestabilisator umfaßt.

5. Stabilisatorzusammensetzung für Polyvinylchloridharz gemäß Anspruch 4, in welcher der Polyvinylchloridharz-Wärmestabilisator ein phenolisches Antioxidationsmittel ist.

6. Stabilisatorzusammensetzung für Polyvinylchloridharz gemäß Anspruch 4, in welcher der Polyvinylchloridharz-Wärmestabilisator ein organisches Phosphit ist.

7. Stabilisatorzusammensetzung für Polyvinylchloridharz gemäß Anspruch 4, in welcher der Polyvinylchloridharz-Wärmestabilisator eine Organozinnverbindung ist.

8. Stabilisatorzusammensetzung für Polyvinylchloridharz gemäß Anspruch 4, in welcher der Polyvinylchloridharz-Wärmestabilisator ein Thioether ist.

9. Polyvinylchloridharzzusammensetzung mit verbesserter Beständigkeit gegen Zersetzung bei Einwirkung von Wärme und Licht, die ein Polyvinylchloridharz und eine Stabilisatorzusammensetzung gemäß Anspruch 4 umfaßt.

**Revendications**

1. Les α-hydroxyéthyl-β-2,2,6,6-tétraméthylpipéridyléthylène oxirannes, qui sont des produits de la réaction d'un composé ayant la formule Ia

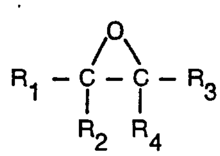

dans laquelle l'un au moins des groupes $R_1$, $R_2$, $R_3$ et $R_4$ inclut un groupe oxiranne, le total de ces groupes oxiranne dans $R_1$, $R_2$, $R_3$ et $R_4$ étant compris dans l'intervalle de 1 à 20 et $R_1$, $R_2$, $R_3$ et $R_4$ étant choisis dans le groupe comprenant l'hydrogène, les groupes hydrocarbonés aliphatiques, aromatiques, aromatiques-aliphatiques-cycloaliphatiques mixtes et cycloaliphatiques-aliphatiques mixtes, ayant de 1 à 30 atomes de carbone et des groupes hétérocycliques ayant de 1 à 3 atomes d'azote et/ou d'oxygène cyclique et de 5 à 3 atomes de carbone cyclique et un composé 2,2,6,6-tétraméthyl-pipéridyle contenant dans la molécule le résidu de formule Ib

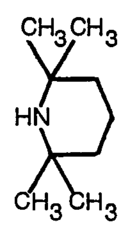

pour produire un composé dans lequel ledit résidu 2,2,6,6-tétraméthylpipéridyle devient lié à un carbone

du noyau oxiranne du composé de formule Ia et l'oxygène du groupe époxy devient un groupe hydroxyle attaché aux autres atomes de carbone du noyau oxiranne.

2. Les α-hydroxy-β-2,2,6,6-tétraméthylpipéridyléthylène oxirannes selon la revendication 1, selon laquelle $R_1$, $R_2$, $R_3$ et $R_4$ sont des groupes hydrocarbonés aliphatiques, dont l'un au moins inclut un groupe oxiranne.

3. Les α-hydroxy-β-2,2,6,6-tétraméthylpipéridyléthylène oxirannes selon la revendication 1, selon laquelle $R_1$, $R_2$, $R_3$ et $R_4$ sont choisis dans le groupe comprenant des groupes hydrocarbonés aliphatiques saturés et insaturés et aliphatiques-aromatiques mixtes et ces groupes incluant au moins un groupe oxiranne.

4. Une composition stabilisante capable d'améliorer la résistance à la détérioration des résines de chlorure de polyvinyle lorsqu'elles sont exposées à la chaleur et à la lumière ultraviolette, comprenant un α-hydroxy-β-2,2,6,6-tétraméthylpipéridyléthylène oxiranne selon une ou plusieurs des revendications 1—3, et un stabilisant thermique de la résine de chlorure de polyvinyle.

5. Une composition stabilisante pour la résine de chlorure de polyvinyle selon la revendication 4, selon laquelle le stabilisant thermique de la résine de chlorure de polyvinyle est un antioxydant phénolique.

6. Une composition stabilisante pour la résine de chlorure de polyvinyle selon la revendication 4, selon laquelle le stabilisant thermique de la résine de chlorure de polyvinyle est un phosphite organique.

7. Une composition stabilisante pour la résine de chlorure de polyvinyle selon la revendication 4, selon laquelle le stabilisant thermique de la résine de chlorure de polyvinyle est un composé organo-étain.

8. Une composition stabilisante pour la résine de chlorure de polyvinyle selon la revendication 4, selon laquelle le stabilisant thermique de la résine de chlorure de polyvinyle est un thioéther.

9. Une composition de résine de chlorure de polyvinyle ayant une résistance améliorée à la détérioration lorsqu'elle est exposée à la chaleur et à la lumière comprenant une résine de chlorure de polyvinyle et une composition stabilisante selon la revendication 4.